# FASCICULE DE BREVET EUROPEEN

(11) **EP 1 378 227 B1**
(45) Date de publication et mention de la délivrance du brevet: **17.01.2007**
(21) Numéro de dépôt: 03291386.5
(22) Date de dépôt: 11.06.2003
(51) Int. Cl.: A61K 8/41, A61K 8/49, A61K 8/73, A61K 8/81, A61K 8/895, A61K 8/896, A61Q 5/12

(54) **Composition cosmétique comprenant une silicone quaternaire, un tensioactif cationique, deux polymères cationiques et procédé de traitement cosmétique**
Kosmetische Zusammensetzung die ein quaternäres Silikon, ein kationisches Tensid, zwei kationische Polymere enthalten, und kosmetische Behandlungsverfahren
Cosmetic composition comprising a quaternary silicone, a cationic surfactant and two cationic polymers, as well as cosmetic treatment process

(30) Priorité: 28.06.2002 FR 0208142
(43) Date de publication de la demande: 07.01.2004
(73) Titulaire: L'ORÉAL, 75008 Paris (FR)
(72) Inventeur: Decoster, Sandrine, 95210 Saint Gratien (FR); Cazin, Bénédicte, 92110 Clichy (FR)
(74) Mandataire: Le Blainvaux Bellegarde, Françoise

(56) Documents cités:
- EP-A- 1 080 714
- WO-A-00/64411
- WO-A-01/28505
- WO-A-02/22084
- DE-C- 19 735 865
- GB-A- 2 165 550

## Description

La présente invention est relative à une composition cosmétique non lavante notamment de conditionnement des cheveux comprenant une silicone à groupements ammonium quaternaire, un cation, au moins un polymère épaississant non ionique et au moins deux polymères cationiques et à un procédé de traitement cosmétique des matières kératiniques en particulier les cheveux.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà proposé pour le traitement des matières kératiniques et en particulier des cheveux, des compositions cosmétiques contenant des polysaccharides épaississants tels que notamment l'amidon ou les celluloses.

De telles compositions présentent cependant des inconvénients tels que des problèmes de rinçabilité, des problèmes de stabilité, des difficultés de répartition sur les matières kératiniques ainsi que des propriétés cosmétiques insuffisantes.

On a déjà préconisé dans les compositions pour le lavage ou le soin des matières kératiniques telles que les cheveux l'utilisation de polymères cationiques, de silicones cationiques ou de tensioactifs cationiques pour faciliter le démêlage des cheveux et pour leur communiquer douceur et souplesse. L'usage des polymères cationiques ou des cations dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage.

En résumé, il s'avère que les compositions cosmétiques actuelles de conditionnement, ne donnent pas complètement satisfaction.

Par ailleurs, on cherche quelquefois à obtenir des compositions cosmétiques transparentes qui sont particulièrement appréciées des consommateurs. Les compositions de conditionnement classiques à base d'alcools gras de l'art antérieur ne sont pas transparentes.

Le document WO 02/220 84 décrit des compositions cosmétiques concentrées pour le soin des cheveux, qui leur confèrent un toucher doux si mouillés, une facilité de peignage, et de la douceur.

La demanderesse a maintenant découvert que l'association d'une silicone quaternaire, d'un tensioactif cationique, d'au moins deux polymères cationiques et d'un polymère épaississant non ionique, notamment dans des milieux non détergents ayant une concentration faible ou nulle en agents tensio-actifs lavants permet de remédier à ces inconvénients.

Les cheveux traités avec cette composition sont lisses, se démêlent facilement sont brillants, souples, individualisés et ont un toucher doux et sans résidus. Les cheveux ont un aspect naturel et non chargé.

En outre, ces compositions sont transparentes et ont une texture fondante, c'est à dire qui disparaît rapidement dans la chevelure.

Ainsi, selon la présente invention, il est maintenant proposé de nouvelles compositions cosmétiques non lavantes , comprenant, dans un milieu cosmétiquement acceptable, au moins une silicone à groupements ammonium quaternaire, au moins un tensioactif cationique, au moins deux polymères cationiques différents l'un de l'autre et au moins un agent épaississant polymérique non ionique et non associatif.

Un autre objet de l'invention consiste en un procédé de traitement cosmétique des matières kératiniques, en particulier les cheveux mettant en oeuvre la composition susvisée.

L'invention a encore pour objet l'utilisation de ladite composition comme après-shampoing.

L'invention a encore pour objet l'utilisation de ladite composition pour apporter de la brillance aux cheveux.

L'invention a encore pour objet l'utilisation de ladite composition pour apporter de la souplesse aux cheveux.

D'autres objets, caractéristiques, aspects et avantages de l'invention apparaîtront encore plus clairement à la lecture de la description et des divers exemples qui suivent.

Conformément à l'invention, on entend par "silicone à groupements ammonium quaternaire" toute silicone comportant un ou plusieurs groupements ammonium quaternaire. Ces groupements ammonium quaternaire peuvent être liés en position alpha ou oméga ou sous forme de groupements latéraux. Ils peuvent être liées directement au squelette polysiloxane ou peuvent être portés par des chaînes hydrocarbonées.

Selon l'invention, on entend par silicone, en conformité avec l'acceptation générale, tout polymère ayant une structure basée sur l'alternance d'atomes de silicium et d'oxygène, reliés entre eux par des liaisons dites liaisons siloxane (-Si-O-Si-), et caractérisée en outre par l'existence de liaisons silicium-carbone. Ces silicones, ou polysiloxanes, sont généralement obtenues par polycondensation de silanes convenablement fonctionnalisés. Les radicaux hydrocarbonés les plus courants portés par les atomes de silicium sont les radicaux alkyle inférieurs, en particulier méthyle, les radicaux fluoroalkyles, les radicaux aryle et en particulier phényle.

Les silicones à groupements ammonium quaternaire de la présente invention sont par exemple choisies parmi les composés correspondants aux formules générales suivantes : 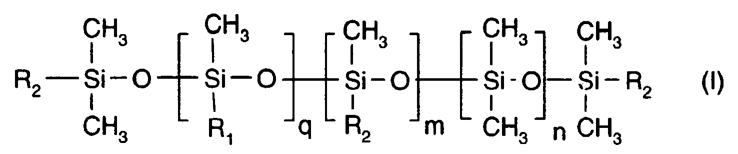 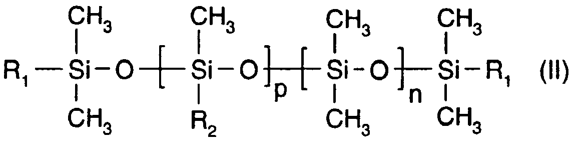 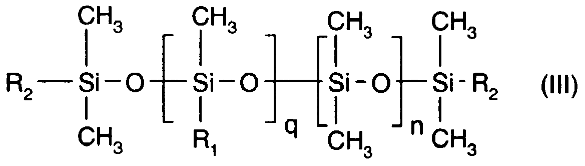 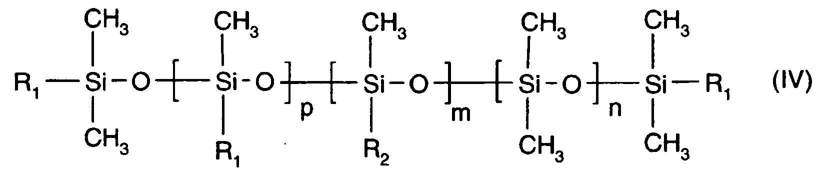 formules dans lesquelles :
- R₁, identique ou différent, représente un groupe alkyle, linéaire ou ramifié, en C₁-C₃₀, ou phényle ;
- R₂, identique ou différent, représente -C_{c}H_{2c}-O-(C₂H₄O)ₐ-(C₃H₆O)_{b}-R₅ ou - C_{c}H_{2c}-O-(C₄H₈O)ₐ-R₅ ;
- R₃ et R₄, identiques ou différents, désignent chacun un groupe alkyle, linéaire ou ramifié, en C₁-C₁₂, et de préférence un groupe méthyle ;
- R₅, identique ou différent, est choisi parmi les groupes de formule suivante : 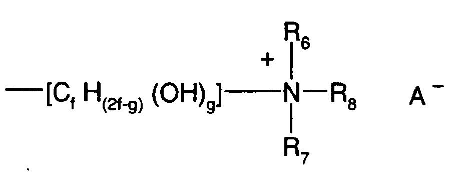
- les radicaux R₈ représentent indépendamment un radical alkyle en C₁₋₂₂ ou alcényle en C₂₋₂₂, linéaire ou ramifié, et portant éventuellement un ou plusieurs groupements OH, ou représentent un groupement CₕH₂ₕZCOR₉;
- R₆, R₇ et R₉, identiques ou différents, représentent des radicaux alkyle en C₁₋₂₂
ou alcényle en C₂₋₂₂, linéaires ou ramifiés, portant éventuellement un ou plusieurs groupements OH, ou R₇ peut former avec une partie de R₈ un hétérocycle (cycle avec au moins un hétéroatome tel que par exemple N, O, P), l'hétéro cycle est notamment une imidazoline. De préférence R₆ et R₇ désignent un radical alkyle en C₁-C₆ et plus particulièrement méthyle, R₉ désigne de préférence un radical choisi parmi les alkyle en C₈-C₁₈ et les alcényl en C₈-C₁₈ et notamment un radical cocoyle.
- m varie de 0 à 20 ;
- n varie de 0 à 500 ;
- p varie de 1 à 50 ;
- q varie de 0 à 20 ;
- a varie de 0 à 50 ;
- b varie de 0 à 50 ;
- c varie de 0 à 4 ;
- f varie de 0 à 4,
- g varie de 0 à 2, de préférence est égal à 1
- h varie de 1 à 4, de préférence est égal à 3
   Z représente un atome d'oxygène ou NH,
A⁻ représente un anion minéral ou organique monovalent tel qu'un halogénure (chlorure, bromure), un sulfate, ou un carboxylate (acétate, lactate, citrate).

De préférence, on utilise les silicones à ammonium quaternaire répondant à la formule générale (III) telle que définie ci-dessus, et plus particulièrement, ceux répondant à la formule générale (III) dans laquelle au moins l'une des, et de préférence toutes les conditions suivantes sont satisfaites :
- c est égal à 2 ou 3 ;
- R₁ désigne le groupe méthyle ;
- a et b sont égaux à zéro ;
- n varie de 0 à 100 ;
- q est égal à 0 ;
- f = 3;
- g = 1 ;
- R₆ et R₇ désignent le groupe méthyle ;
- R₈ désigne le radical -(CH₂)-NHCOR₉.

De telles silicones sont commercialisées par exemple par la société GOLDSCHMIDT sous les dénominations ABIL QUAT 3272, ABIL B 9905, ABIL QUAT 3474 et ABIL K 3270, par la société LIPO FRANCE sous les dénominations SILQUAT Q-100, SILQUAT Q-200 WS, SILQUAT AX, SILQUAT AC, SILQUAT AD et SILQUAT AM tous fabriqués par la société SILTECH, par la société OSI sous la dénomination MAGNASOFT EXHAUST et SILSOFT C-880 et par la société UCIB sous les dénominations PECOSIL 14-PQ et PECOSIL 36-PQ (fabricant PHOENIX CHEMICAL).

Ces silicones sont notamment décrites dans les brevets EP 530 974, DE 3 719 086, DE 3 705 121, EP 617 607 et EP 714 654.

Les silicones à groupements ammonium quaternaire utilisées conformément à l'invention peuvent se présenter sous forme de solutions aqueuses ou éventuellement sous forme de dispersions ou d'émulsions dans l'eau.

Dans les compositions de la présente invention, la ou les silicones à groupements ammonium quaternaire sont présentes à raison de 0,01 à 10 % en poids, de préférence à raison de 0,1 à 5 % en poids de matière active , plus particulièrement de 0,1 à 1% en poids par rapport au poids total de la composition.

La composition selon l'invention comprend un ou plusieurs tensioactifs cationiques bien connus en soi, tels que les sels d'amines grasses primaires, secondaires ou tertiaires, éventuellement polyoxyalkylénées, les sels d'ammonium quaternaire, et leurs mélanges.

A titre de sels d'ammonium quaternaire, on peut notamment citer, par exemple :
- ceux qui présentent la formule générale (V) suivante : 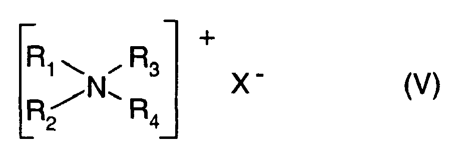
dans laquelle les symboles R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle, alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X⁻ est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline comme, par exemple, ceux de formule (VI) suivante : 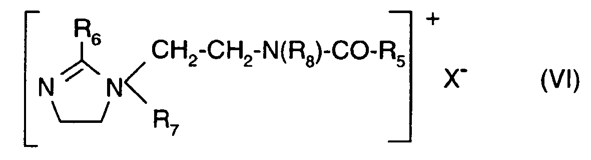 dans laquelle R₅ représente un radical alcényle ou alkyle comportant de 8 à 30 atomes de carbone par exemple dérivés des acides gras du suif ou du coprah, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT®" W 75, W90, W75PG, W75HPG par la société WITCO,
- les sels de diammonium quaternaire de formule (VII) : 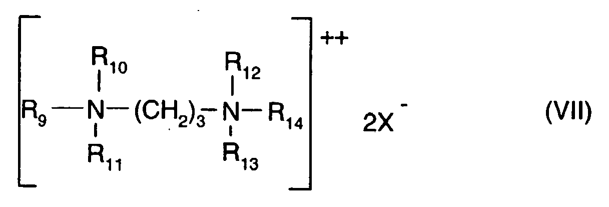 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X⁻ est un anion choisi dans le groupe des halogénures, acétates, phosphates, nitrates, éthylsulfates et méthylsulfates. De tels sels de diammonium quaternaire comprennent notamment le dichlorure de propanesuif diammonium ;
- les sels d'ammonium quaternaire contenant au moins une fonction ester, tels que ceux de formule (VIII) suivante : 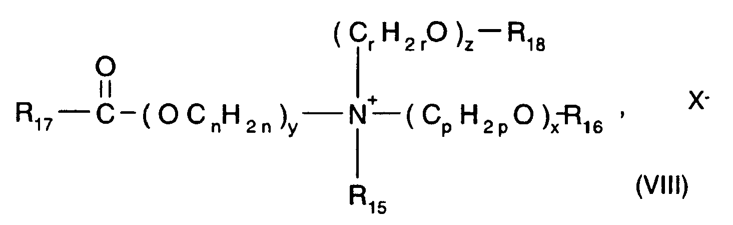
dans laquelle :
R₁₅ est choisi parmi les radicaux alkyles en C₁-C₆ et les radicaux hydroxyalkyles ou dihydroxyalkyles en C₁-C₆;
R₁₆ est choisi parmi :
   - le radical 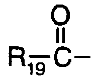
   - les radicaux R₂₀ hydrocarbonés en C₁-C₂₂, linéaires ou ramifiés, saturés ou insaturés,
   - l'atome d'hydrogène,
R₁₇ est choisi parmi :
   - le radical 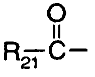
   - les radicaux R₂₂ hydrocarbonés en C₁-C₆, linéaires ou ramifiés, saturés ou insaturés,
   - l'atome d'hydrogène,
R₁₇, R₁₉ et R₂₁, identiques ou différents, sont choisis parmi les radicaux hydrocarbonés en C₇-C₂₁, linéaires ou ramifiés, saturés ou insaturés ;
r, n et p, identiques ou différents, sont des entiers valant de 2 à 6 ;
y est un entier valant de 1 à 10 ;
x et z, identiques ou différents, sont des entiers valant de 0 à 10 ;
X⁻ est un anion simple ou complexe, organique ou inorganique ;
sous réserve que la somme x + y + z vaut de 1 à 15, que lorsque x vaut 0 alors R₁₆ désigne R₂₀ et que lorsque z vaut 0 alors R₁₈ désigne R₂₂.
Les radicaux alkyles R₁₅ peuvent être linéaires ou ramifiés, et plus particulièrement linéaires.
De préférence, R₁₅ désigne un radical méthyle, éthyle, hydroxyéthyle ou dihydroxypropyle, et plus particulièrement un radical méthyle ou éthyle.
Avantageusement, la somme x + y + z vaut de 1 à 10.
Lorsque R₁₆ est un radical R₂₀ hydrocarboné, il peut être long et avoir de 12 à 22 atomes de carbone, ou court et avoir de 1 à 3 atomes de carbone.
Lorsque R₁₈ est un radical R₂₂ hydrocarboné, il a de préférence 1 à 3 atomes de carbone. Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.
De préférence, x et z, identiques ou
différents, valent 0 ou 1.
Avantageusement, y est égal à 1.
De préférence, r, n et p, identiques ou différents, valent 2 ou 3, et encore plus particulièrement sont égaux à 2.

L'anion X⁻ est de préférence un halogénure (chlorure, bromure ou iodure) ou un alkyl(C1-C4)sulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.
L'anion X⁻ est encore plus particulièrement le chlorure ou le méthylsulfate.
On utilise plus particulièrement dans la composition selon l'invention, les sels d'ammonium de formule (IV) dans laquelle :
- R₁₅ désigne un radical méthyle ou éthyle,
- x et y sont égaux à 1 ;
- z est égal à 0 ou 1 ;
- r, n et pt sont égaux à 2 ;
- R₁₆ est choisi parmi :

- le radical 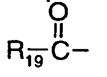
- les radicaux méthyle, éthyle ou hydrocarbonés en C₁₄-C₂₂,
- l'atome d'hydrogène ;
- R₁₈ est choisi parmi:

- le radical 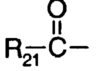
- l'atome d'hydrogène ;
- R17, R₁₉ et R₂₁, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.
Avantageusement, les radicaux hydrocarbonés sont linéaires.

On peut citer par exemple les composés de formule (VIII) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.
Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART® par la société COGNIS, STEPANQUAT® par la société STEPAN, NOXAMIUM® par la société CECA, REWOQUAT® WE 18 par la société REWO-GOLDSCHMIDT.

La composition selon l'invention peut contenir de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire mentionnés ci-dessus, on préfère utiliser ceux répondant à la formule (V). On peut notamment citer d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le chlorure de palmitylamidopropyltriméthylammonium ou le chlorure de stéaramidopropyldiméthyl-(myristyl acétate)-ammonium commercialisé sous la dénomination CERAPHYL® 70 par la société VAN DYK.

Les tensioactifs cationiques particulièrement préférés dans la composition de l'invention sont choisis parmi les sels d'ammonium quaternaire, et en particulier parmi le chlorure de béhényltriméthylammonium, le chlorure de cétyltriméthylammonium, le quaternium-83, le chlorure de béhénylamidopropyl 2,3-dihydroxypropyl diméthyl ammonium et le chlorure de palmitylamidopropyltriméthylammonium.

Les tensioactifs cationiques préférés sont les tensioactifs cationiques solubles dans la composition et en particulier ceux solubles dans l'eau, ou ceux solubilisés dans l'eau ou dans la composition par au moins un tensioactif non ionique.

Par tensioactifs cationiques solubles dans l'eau ou la composition, on entend les tensioactifs cationiques solubles dans l'eau ou la composition à une concentration supérieure ou égale à 0,1% en poids dans l'eau à 25°C, c'est à dire qu'ils forment dans ces conditions une solution macroscopiquement isotrope transparente.

La composition selon l'invention contient de préférence le ou les tensioactifs cationiques en une quantité allant de 0,05 à 10 % en poids, de préférence de 0,1 à 5 % en poids par rapport au poids total de la composition et plus particulièrement de 0,2 à 2% en poids et encore plus préférentiellement de 0,3 à 1 % en poids.

Les compositions selon l'invention comprennent en outre au moins deux polymères cationiques différents l'un de l'autre.

Les polymères cationiques utilisables conformément à la présente invention peuvent être choisis parmi tous ceux déjà connus en soi comme améliorant les propriétés cosmétiques, à savoir notamment ceux décrits dans la demande de brevet EP-A- 0 337 354 et dans les demandes de brevets français FR-A- 2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les homopolymères ou copolymères dérivés d'esters ou d'amides acryliques ou méthacryliques et comportant au moins un des motifs de formules suivantes: 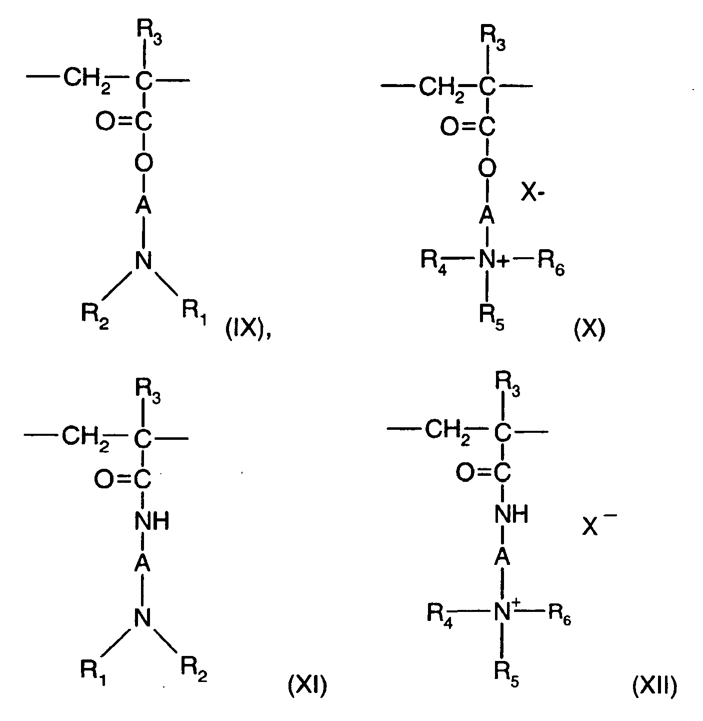 dans lesquelles:
   R₁ et R₂ , identiques ou différents, représentent hydrogène ou un groupe alkyle ayant de 1 à 6 atomes de carbone et de préférence méthyle ou éthyle;
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   X- désigne un anion dérivé d'un acide minéral ou organique tel que un anion méthylsulfate, éthylsulfate ou un halogénure tel que chlorure ou bromure.

Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
Ainsi, parmi ces copolymères de la famille (1), on peut citer :
- les copolymères d'acrylamide et de diméthylaminoéthyl méthacrylate quaternisé au sulfate de diméthyle ou avec un hologénure de diméthyle tels que celui vendu sous la dénomination HERCOFLOC par la société HERCULES,
- les copolymères d'acrylamide et de chlorure de méthacryloyloxyéthyltriméthyl-ammonium décrit par exemple dans la demande de brevet EP-A-080976 et vendus sous la dénomination BINA QUAT P 100 par la société CIBA ,
- le copolymère d'acrylamide et de méthosulfate de méthacryloyloxyéthyltriméthyl-ammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT® 734" ou "GAFQUAT® 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/ vinylpyrrolidone tel que le produit vendu sous la dénomination GAFFIX® VC 713 par la société ISP,
- les copolymères vinylpyrrolidone / méthacrylamidopropyl dimethylamine commercialisés notamment sous la dénomination STYLEZE® CC 10 par ISP.
- et les copolymères vinylpyrrolidone / méthacrylamide de diméthylaminopropyle quaternisé tel que le produit vendu sous la dénomination "GAFQUAT® HS 100" par la société ISP.

2)Les polysaccharides cationiques notamment les celluloses, les amidons et les gommes de galactomannanes cationiques. Parmi les polysaccharides cationiques, on peut citer plus particulièrement les éthers de cellulose comportant des groupements ammonium quaternaires, les copolymères de cellulose cationiques ou les celluloses greffées avec un monomère hydrosoluble d'ammonium quaternaire et les gommes de galactomannanes cationiques.

Les éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la société AMERCHOL. Ces polymères sont également définis dans le dictionnaire CTFA comme des ammonium quaternaires d'hydroxyéthylcellulose ayant réagi avec un époxyde substitué par un groupement triméthylammonium.

Les copolymères de cellulose cationiques ou les celluloses greffées avec un monomère hydrosoluble d'ammonium quaternaire, sont décrits notamment dans le brevet US 4 131 576. On peut notamment citer les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.

Les produits commercialisés répondant à cette définition sont plus particulièrement les produits vendus sous la dénomination "CELQUAT® L 200" et "CELQUAT® H 100" par la société NATIONAL STARCH.

Les gommes de galactomannane cationiques sont décrites plus particulièrement dans les brevets US 3 589 578 et 4 031 307 en particulier les gommes de guar contenant des groupements cationiques trialkylammonium. On utilise par exemple des gommes de guar modifiées par un sel (par ex. chlorure) de 2,3-époxypropyl triméthylammonium.

De tels produits sont commercialisés notamment sous les dénominations commerciales de JAGUAR® C13 S, JAGUAR® C 15, JAGUAR® C 17 ou

JAGUAR® C162 par la société RHODIA CHIMIE.

On utilise également des amidons modifiées par un sel (par ex. chlorure) de 2,3-époxypropyl triméthylammonium tels que par exemple le produit dénommé Starch hydroxypropyltrimonium chloride selon la nomenclature INCI et commercialisé sous la dénomination SENSOMER CI-50 d'ONDEO.
(3)les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(5) les polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.

Parmi ces dérivés, on peut citer plus particulièrement les polymères acide adipique/diméthylaminohydroxypropyl/diéthylène triamine vendus sous la dénomination "CARTARETINE® F, F4 ou F8" par la société SANDOZ.
(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.

Des polymères de ce type sont en particulier commercialisés sous la dénomination "HERCOSETT® 57" par la société HERCULES Inc. ou bien sous la dénomination de "PD 170" ou "DELSETTE® 101" par la société HERCULES dans le cas du copolymère d'acide adipique/époxypropyl/diéthylène-triamine.
(7)les cyclopolymères d'alkyl diallyl amine ou de dialkyl diallyl ammonium tels que les homopolymères ou copolymères comportant comme comprenant principal de la chaîne des motifs répondant aux formules (XIII) ou (XIV) : 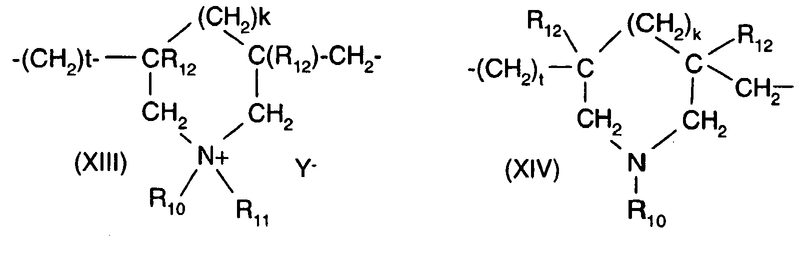 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 8 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.

Parmi les polymères définis ci-dessus, on peut citer plus particulièrement l'homopolymère de chlorure de diméthyldiallylammonium vendu sous la dénomination "MERQUAT®) 100" par la société NALCO (et ses homologues de faibles masses moléculaires moyenne en poids) et les copolymères de chlorure de diallyldiméthylammonium et d'acrylamide commercialisés sous la dénomination "MERQUAT® 550".
(8) les polymères de diammonium quaternaires contenant des motifs récurrents répondant à la formule : 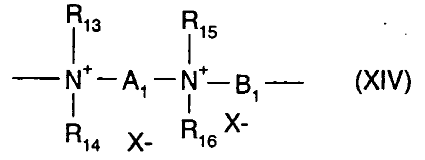 formule (XIV) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄,
   R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C₁-C₆ linéaire ou ramifié substitué par un groupement nitrile, ester, acyle, amide ou -CO-O-R₁₇-D ou -CO-NH-R₁₇-D où R₁₇ est un alkylène et
   D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
   dans lequel D désigne :
   a)un reste de glycol de formule : -O-Z-O-, où Z désigne un radical hydrocarboné linéaire ou ramifié ou un groupement répondant à l'une des formules suivantes : -(CH2-CH2-O)x-CH2-CH2--[CH2-CH(CH3)-O]y-CH2-CH(CH3)-
      où x et y désignent un nombre entier de 1 à 4, représentant un degré de polymérisation défini et unique ou un nombre quelconque de 1 à 4 représentant un degré de polymérisation moyen ;
   b)un reste de diamine bis-secondaire tel qu'un dérivé de pipérazine ;
   c)un reste de diamine bis-primaire de formule : -NH-Y-NH-, où Y désigne un radical hydrocarboné linéaire ou ramifié, ou bien le radical bivalent -CH2-CH2-S-S-CH2-CH2- ;
   d)un groupement uréylène de formule : -NH-CO-NH- ;

De préférence, X⁻ est un anion minéral ou organique monovalent tel qu'un halogénure (chlorure, bromure), un sulfate, ou un carboxylate (acétate, lactate, citrate).

Ces polymères ont une masse moléculaire moyenne en nombre généralement comprise entre 1.000 et 100.000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule : 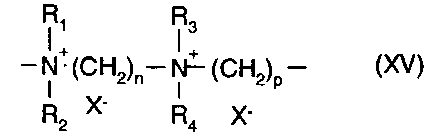 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
Un composé de formule (XV) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).
(9) les polymères de polyammonium quaternaires constitués de motifs de formule (XVI): 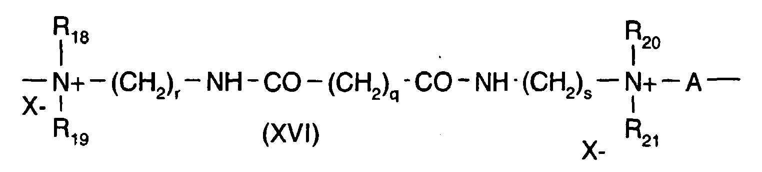 formule dans laquelle :
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
      où p est égal à 0 ou à un nombre entier allant de 1 et 6, sous réserve que R₁₈, R₁₉, R₂₀ et R₂₁ ne représentent pas simultanément un atome d'hydrogène,
   r et s, identiques ou différents, sont des nombres entiers allant de 1 et 6,
   q est égal à 0 ou à un nombre entier allant de 1 et 34,
   X⁻ désigne un anion tel qu'un halogènure,
   A désigne un radical d'un dihalogénure ou représente de préférence -CH₂-CH₂-O-CH₂-CH₂-.

De tels composés sont notamment décrits dans la demande de brevet EP-A-122 324.

On peut par exemple citer parmi ceux-ci, les produits "MIRAPOL® A 15", "MIRAPOL® AD1", "MIRAPOL® AZ1" et "MIRAPOL® 175" vendus par la société MIRANOL.
(11)Les polymères quaternaires de vinylpyrrolidone et de vinylimidazole tels que les polyquaternium-11, polyquaternium-16, polyquaternium-44 notamment les produits commercialisés sous les dénominations LUVIQUAT® FC 905, FC 550, FC 370 et LUVIQUAT® CARE par la société B.A.S.F.
(11) Les polyamines comme le POLYQUART® H vendu par COGNIS, référencé sous le nom de « POLYETHYLENEGLYCOL (15) TALLOW POLYAMINE » dans le dictionnaire CTFA.
(12) Les polymères réticulés ou non réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE® SC 95 » et « SALCARE® SC 96 » par la société CIBA.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.
Parmi tous les polymères cationiques susceptibles d'être utilisés dans le cadre de la présente invention, on préfère mettre en oeuvre les polysaccharides cationiques notamment les dérivés d'éther de cellulose quaternaires tels que les produits vendus sous la dénomination « JR 400 » par la société AMERCHOL, les cyclopolymères cationiques, en particulier les homopolymères ou copolymères de chlorure de diméthyldiallylammonium, vendus sous les dénominations « MERQUAT® 100 », « MERQUAT® 550 » et « MERQUAT® S » par la société NALCO, les copolymères de vinylpyrrolidone et de sels (par ex méthylsulfate ou éthylsulfate) de méthyl vinylimidazolium notamment vendus sous la dénomination LUVIQUAT CARE par BASF et leurs mélanges.

Selon l'invention, la composition comprend de préférence au moins un polysaccharide cationique et au moins un copolymère de vinylpyrrolidone en particulier les copolymères quaternaires de vinylpyrrolidone et de vinylimidazole.

Selon l'invention, la composition comprend de préférence au moins un polymère de chlorure de diallyldiméthylammonium de préférence un homopolymère et au moins un copolymère de vinylpyrrolidone en particulier les copolymères quaternaires de vinylpyrrolidone et de vinylimidazole.

Selon l'invention, chaque polymère cationique peut représenter de 0,001 % à 20 % en poids, de préférence de 0,01 % à 10% en poids, de 0,05 à 5% en poids et plus particulièrement de 0,1 à 3% en poids par rapport au poids total de la composition finale et encore plus particulièrement de 0,1 à 1% en poids.

Selon l'invention, la concentration totale en polymère cationique peut représenter de 0,01 % à 20 % en poids, de préférence de 0,05 % à 5% en poids, plus particulièrement de 0,1 à 3% en poids par rapport au poids total de la composition finale et encore plus particulièrement de 0,5 à 1,5% en poids.

Par l'expression "polymère épaississant non associatif", on entend selon l'invention, un polymère épaississant ne comportant pas à la fois au moins une chaîne grasse en C8-C30 et au moins un motif hydrophile.

Les polymères épaississants non ioniques non associatifs selon l'invention peuvent être d'origine naturelle ou synthétique. Ils sont notamment choisis parmi:
(i) les homopolymères et copolymères non-ioniques contenant des monomères à insaturation éthylénique de type ester et/ou amide,
(ii)les homo ou copolymères de vinylpyrrolidone,
(iii) les polysaccharides.

Parmi les homopolymères ou copolymères non-ioniques contenant des monomères à insaturation éthylénique de type ester et/ou amide, on peut citer les polyamides notamment les produits vendus sous les dénominations de : CYANAMER P250 par la société CYTEC (polyacrylamide); les copolymères méthacrylate de méthyle / diméthacrylate d'éthylèneglycol ( PMMA MBX-8C par la société US COSMETICS ) ; les copolymères méthacrylate de butyle / méthacrylate de méthyle (ACRYLOID B66 par la société RHOM & HAAS), les polyméthacrylate de méthyle (BPA 500 par la société KOBO);

Les homo ou copolymères de vinylpyrrolidone sont notamment choisis parmi les homopolymères de vinylpyrrolidone réticulés tels que le POLYMER ACP-10 commercialisé par ISP;

Les polysaccharides épaississant sont notamment choisis parmi les glucanes, les amidons modifiés ou non (tels que ceux issus, par exemple, de céréales comme le blé, le maïs ou le riz, de légumes comme le pois blond, de tubercules comme les pommes de terre ou le manioc), l'amylose, l'amylopectine, le glycogène les dextranes, les celluloses et leurs dérivés (méthylcelluloses, hydroxyalkylcelluloses, éthylhydroxyéthylcelluloses), les mannanes, les xylanes, les lignines, les arabanes, les galactanes, les galacturonanes, la chitine, les chitosanes, les glucoronoxylanes, les arabinoxylanes, les xyloglucanes, les glucomannanes, les acides pectiques et les pectines, les arabinogalactanes, les carraghénines, les agars, les gommes arabiques, les gommes Tragacanthe, les gommes Ghatti, les gommes Karaya, les gommes de caroube, les galactomannanes telles que les gommes de guar et leurs dérivés non ioniques (hydroxypropyl guar), et leurs mélanges.

D'une manière générale, les composés de ce type, utilisables dans la présente invention, sont choisis parmi ceux qui sont notamment décrits dans "Encyclopedia of Chemical Technology, Kirk-Othmer, Third Edition, 1982, volume 3, pp. 896-900, et volume 15, pp 439-458", dans "Polymers in Nature, par E. A. MacGREGOR et C. T. GREENWOOD, Editions John Wiley & Sons, Chapter 6, pp 240-328, 1980" et dans l'Industrial Gums - Polysaccharides and their Derivatives, Edité par Roy L. WHISTLER, Second Edition, Edition Academic Press Inc.", le contenu de ces trois ouvrages étant totalement inclus dans la présente demande à titre de référence.
On utilisera de préférence, les amidons, les gommes de guar, les celluloses et leurs dérivés.

Les polysaccharides peuvent être modifiées ou non modifiées.
Les gommes de guar non modifiées sont par exemple les produits vendus sous la dénomination VIDOGUM GH 175 par la société UNIPECTINE, et sous les dénominations MEYPRO-GUAR 50 et JAGUAR C par la société RHODIA CHIMIE.
Les gommes de guar non-ioniques modifiées sont notamment modifiées par des groupements hydroxyalkyle en C₁-C₆.
Parmi les groupements hydroxyalkyle, on peut mentionner à titre d'exemple, les groupements hydroxyméthyle, hydroxyéthyle, hydroxypropyle et hydroxybutyle.
Ces gommes de guar sont bien connues de l'état de la technique et peuvent, par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.
Le taux d'hydroxyalkylation, qui correspond au nombre de molécules d'oxyde d'alkylène consommées par le nombre de fonctions hydroxyle libres présentes sur la gomme de guar, varie de préférence de 0,4 à 1,2.
De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société RHODIA CHIMIE ou sous la dénomination GALACTASOL 4H4FD2 par la société AQUALON.

Parmi les celluloses on utilise notamment les hydroxyéthyl celluloses, les hydroxypropylcelluloses. On peut citer les produits vendus sous les dénominations KLUCEL EF, KLUCEL H, KLUCEL LHF, KLUCEL MF, KLUCEL G, par la société AQUALON, le CELLOSIZE POLYMER PCG-10 par la société AMERCHOL.

De préférence, les polymères épaississants des compositions cosmétiques conformes à la présente invention présentent avantageusement en solution ou en dispersion, à 1 % de matière active dans l'eau, une viscosité mesurée au moyen d'un viscosimètre Brookfield à une vitesse de cisaillement de 20T/mn, supérieure à 5 mPa/s, et plus avantageusement encore supérieure 10 mPa/s.

Selon l'invention, le ou les polymères épaississants peuvent représenter de 0,001 % à 20 % en poids, de préférence de 0,01 % à 10% en poids et plus particulièrement de 0,1 à 3% en poids par rapport au poids total de la composition finale et encore plus particulièrement de 0,2 à 2% en poids.

La composition selon l'invention peut contenir éventuellement d'autres tensioactifs que les tensioactifs cationiques.

Les tensioactifs utilisables dans la présente invention sont choisis parmi les tensioactifs anioniques, non ioniques et amphotères classiques bien connus dans la technique, et leurs mélanges.

Les tensioactifs sont généralement présenten une quantité comprise entre 0,1% et 10% en poids environ, de préférence entre 0,5% et 8% et encore plus préférentiellement entre 1 % et 5%, par rapport au poids total de la composition.

La composition selon l'invention contient de préférence au moins un tensioactif choisi parmi les tensioactifs non ioniques.

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools gras polyéthoxylés, polypropoxylés ou polyglycérolés, les alpha-diols gras polyéthoxylés, polypropoxylés ou polyglycérolés, les alkylphénols gras polyéthoxylés, polypropoxylés ou polyglycérolés, ou les acides gras polyéthoxylés, polypropoxylés ou polyglycérolés, tous ces composés ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

La concentration des tensioactifs non ioniques dans les compositions non détergentes peut varier de 0,1 à 10% en poids et de préférence de 0,5 à 8% en poids et plus particulièrement de 1 à 5% en poids par rapport au poids total de la composition.

Les compositions non lavantes (non détergentes) comprennent de préférence moins de 3% en poids de tensioactifs détergents notamment anioniques et plus particulièrement moins de 1 % en poids par rapport au poids total de la composition.

Les compositions non lavantes (non détergentes) comprennent de préférence moins de 3% en poids de tensioactifs anioniques et plus particulièrement moins de 1% en poids par rapport au poids total de la composition.

La composition selon l'invention peut comprendre en outre au moins un agent conditionneur choisi parmi les silicones différentes des silicones à groupement ammonium quaternaire, les esters carboxyliques comprenant au moins 12 atomes de carbone, les huiles végétales, les huiles minérales, les huiles de synthèse telles que les poly(alpha-oléfines), et leurs mélanges.

Les silicones utilisables conformément à l'invention peuvent être solubles ou insolubles dans la composition, et elles peuvent être en particulier des polyorganosiloxanes insolubles dans la composition de l'invention. Elles peuvent se présenter sous forme d'huiles, de cires, de résines ou de gommes. Elles peuvent être utilisées pures ou en émulsion, en dispersion ou en microémulsion.

Les organopolysiloxanes sont définis plus en détail dans l'ouvrage de Walter NOLL "Chemistry and Technology of Silicones" (1968) Academic Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et, de préférence, 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.

On peut également citer les cyclocopolymères du type diméthylsiloxane/méthylalkylsiloxane, tel que la "SILICONE VOLATILE FZ 3109" commercialisée par la société UNION CARBIDE de structure chimique :

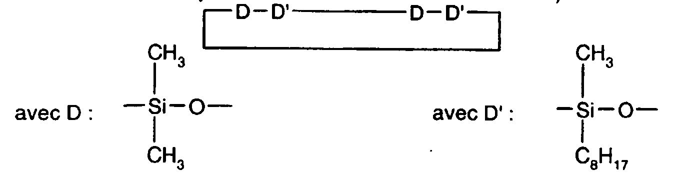

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Parmi les silicones non volatiles, on peut notamment citer les polyalkylsiloxanes, les polyarylsiloxanes, les polyalkylarylsiloxanes, les gommes et les résines de silicones, les polyorganosiloxanes modifiés par des groupements organofonctionnels ainsi que leurs mélanges.

Les silicones organomodifiées utilisables conformément à l'invention sont des silicones telles que définies précédemment et comportant dans leur structure un
ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET® L 722, L 7500, L 77, L 711 de la société UNION CARBIDE et l'alkyl(C₁₂)-méthicone-copolyol commercialisée par la société DOW CORNING sous la dénomination Q2 5200 ;
- des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 ;
- des groupements acyloxyalkyle tels que, par exemple, les polyorganosiloxanes décrits dans le brevet US-A-4957732 ;
- des groupements anioniques du type acide carboxylique comme, par exemple, dans les produits décrits dans le brevet EP 186 507 de la société CHISSO CORPORATION, ou du type alkyl-carboxylique comme ceux présents dans le produit X-22-3701 ^{E} de la société SHIN-ETSU ; 2-hydroxyalkylsulfonate ; 2-hydroxyalkylthiosulfate tels que les produits commercialisés par la société GOLDSCHMIDT sous les dénominations "ABIL® S201" et "ABIL® S255" ;
- des groupements hydroxyacylamino, comme les polyorgano-siloxanes décrits dans la demande EP 342 834. On peut citer, par exemple, le produit Q2-8413 de la société DOW CORNING.

A titre d'exemples de silicones, on utilise de préférence les polydiméthylsiloxanes, les polyalkylarylsiloxanes, les polydiméthylsiloxanes à groupements aminés ou alcoxylés.

La composition selon l'invention peut également comprendre un ou plusieurs esters d'acide carboxylique, tels que par exemple, les composés de formule RₐCOOR_{b} dans laquelle Rₐ représente le reste d'un acide gras supérieur comportant de 4 à 29 atomes de carbone et R_{b} représente une chaîne hydrocarbonée contenant de 3 à 30 atomes de carbone, telles que l'huile de Purcellin (octanoate de stéaryle), le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le laurate de 2-hexyldécyle, le palmitate de 2-octyldécyle, le myristate ou le lactate de 2-octyl-dodécyle, le néopentanoate d'isostéaryle et leurs mélanges.

La composition selon l'invention peut encore comprendre une ou plusieurs huiles végétales telles que l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum et leurs mélanges.

Comme huiles minérales, on peut notamment citer l'huile de paraffine et l'huile de vaseline.

Les agents conditionneurs, choisis parmi les silicones, les esters, les huiles végétales, les huiles minérales, les huiles de synthèse et leurs mélanges, sont contenus de préférence dans la composition selon l'invention en une quantité allant de 0,01 % à 20 % en poids, mieux encore allant de 0,1 % à 10 % en poids et plus particulièrement allant de 0,3 % à 5 % en poids par rapport au poids total de la composition.

Le milieu cosmétiquement acceptable est de préférence aqueux et peut être comprendre de l'eau ou un mélange d'eau et d'un solvant cosmétiquement acceptable tel qu'un alcool inférieur en C₁-C₄, par exemple l'éthanol, l'isopropanol, le tertio-butanol, le n-butanol ; les polyols comme le propylèneglycol, la glycérine ; les éthers de polyols ; les alcanes en C₅-C₁₀; l'acétone, la méthyléthylcétone ; les acétates d'alkyle en C₁-C₄ comme l'acétate de méthyle, l'acétate d'éthyle, l'acétate de butyle ; le diméthoxyéthane, le diéthoxyéthane ; et leurs mélanges. Les solvants sont de préférence choisis parmi la glycérine et le propylène glycol.

Le milieu cosmétiquement acceptable notamment aqueux représente de 30 à 98% en poids par rapport au poids total de la composition.

Les solvants sont de préférence présents dans des concentrations allant de 0,5 à 30% en poids par rapport au poids total de la composition.

Le pH des compositions de l'invention est compris entre 2 et 8, de préférence entre 3 et 7.

Selon l'invention, les compositions sont de préférence transparentes.
La transparence peut se mesurer par la turbidité au turbidimètre HACH - Modèle 2100 P à 25°C (L'appareil est étalonné avec de la formazine). La turbidité des compositions selon l'invention (en l'absence de composés additionnels insolubles) est alors généralement comprise entre 0,05 et 500 NTU et de préférence entre 10 et 300 NTU.

Les compositions selon l'invention peuvent également contenir des additifs classiques bien connus dans la technique, tels que des polymères anioniques, non ioniques ou amphotères, des épaississants non polymériques comme des acides ou des électrolytes, des opacifiants, des nacrants, des vitamines, des provitamines telles que le panthénol, des cires telles que les cires végétales, des céramides naturels ou synthétiques, des parfums, des colorants, des particules organiques ou minérales, des conservateurs, des agents de stabilisation du pH.

L'homme de métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 20 % en poids par rapport au poids total de la composition.

Les compositions de l'invention peuvent se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Elles peuvent être utilisées, par exemple, comme après-shampoings, soins, masques de soin profond, lotions ou crèmes de traitement du cuir chevelu. Ces compositions peuvent être rincées ou non rincées.

Selon un mode de réalisation préféré de l'invention, la composition peut être utilisée comme après-shampoing, en particulier sur des cheveux fins. Cet après-shampooing peut être rincé ou non rincé et de préférence rincé.

Les compositions cosmétiques selon l'invention peuvent se présenter sous forme de gel, de lait, de crème, d'émulsion, de lotion fluides ou épaissies ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

La présente invention concerne également un procédé de traitement cosmétique des matières kératiniques telles que par exemple la peau ou les cheveux qui consiste à appliquer une quantité efficace d'une composition cosmétique telle que décrite ci-dessus, sur les matières kératiniques, à effectuer un éventuel rinçage après un éventuel temps de pose.
Le rinçage s'effectue par exemple avec de l'eau.

Ainsi, ce procédé selon l'invention permet le maintien de la coiffure, le traitement, le conditionnement, le soin des cheveux ou de toute autre matière kératinique.

Les exemples suivants illustrent la présente invention et ne doivent être considérés en aucune manière comme limitant l'invention.

### EXEMPLE 1

On a préparé la composition suivante d'après-shampooing à rincer :

| | |
|---|---|
| Chlorure de cétyl triméthyl ammonium ( DEHYQUART A OR de COGNIS) | 0,8 gMA |
| Quaternium-80 en solution à 50% MA dans le propylène glycol (ABIL QUAT 3272 de GOLDSCHMIDT) | 0,5 gMA |
| Hydroxypropylguar (JAGUAR HP 105 de RHODIA CHIMIE) | 0,4 g |
| Polyquaternium-10 (JR400 de RHODIA CHIMIE) | 0,5 g |
| Polyquaternium-44 (LUVIQUAT CARE de BASF) | 0,57 gMA |
| Hydroxyéthylcellulose (CELLOSIZE POLYMER PCG-10 de UNION CARBIDE ) | 0,3g |
| Glycérine | 5 g |
| Mono laurate de sorbitane oxyéthyléné (20 OE) (TWEEN 20 de UNIQEMA) | 0,4 g |
| Acide citrique | 0,4g |
| Parfum | qs |
| Conservateurs | qs |
| Eau qsp | 100 g |

Dans une cuve de fabrication, on introduit l'eau à température ambiante puis on ajoute les conservateurs et le polyquaternium-10. On homogénéise jusqu'à dissolution complète. Puis on ajoute successivement, en homogénéisant entre chaque addition, l'hydroxypropyl guar, l'hydroxyéthyl cellulose préalablemement dispersée dans la glycérine, puis l'acide citrique, le chlorure de cétyl triméthyl ammonium, le polyquaternium-44 et le quaternium-80.
Quand le mélange est homogène, on ajoute le parfum préalablement dispersé dans le mono laurate de sorbitane oxyéthyléné (20 OE).

La composition a été appliquée pendant 1 à 5 minutes sur les cheveux lavés et essorés. On a ensuite rincé et séché les cheveux.
Les cheveux mouillés sont alors lisses et souples et les cheveux séchés sont malléables et individualisés.

### EXEMPLE 2

On a préparé la composition suivante d'après-shampooing à rincer :

| | |
|---|---|
| Chlorure de cétyl triméthyl ammonium ( DEHYQUART A OR de COGNIS) | 0,8 gMA |
| Quaternium-80 en solution à 50% MA dans le propylène glycol (ABIL QUAT 3272 de GOLDSCHMIDT) | 0,5 gMA |
| Hydroxypropylguar (JAGUAR HP 105 de RHODIA CHIMIE) | 0,2 g |
| Polyquaternium-10 (JR400 de RHODIA CHIMIE) | 0,5 g |
| Polyquaternium-44 (LUVIQUAT CARE de BASF) | 0,57 gMA |
| Hydroxyéthylcellulose (CELLOSIZE POLYMER PCG-10 de UNION CARBIDE ) | 0,7 g |
| Glycérine | 5 g |
| Propylène glycol | 2 g |
| Acide citrique | 0,5 g |
| Parfum | qs |
| Conservateurs | qs |
| Eau qsp | 100 g |

La composition a été appliquée pendant 1 à 5 minutes sur les cheveux lavés et essorés. On a ensuite rincé et séché les cheveux.
Les cheveux mouillés sont alors lisses et souples et les cheveux séchés sont brillants, souples et individualisés.

### EXEMPLE 3

On a préparé la composition suivante d'après-shampooing à rincer :

| | |
|---|---|
| Chlorure de cétyl triméthyl ammonium ( DEHYQUART A OR de COGNIS) | 0,8 gMA |
| Quaternium-80 en solution à 50% MA dans le propylène glycol (ABIL QUAT 3272 de GOLDSCHMIDT) | 0,5 gMA |
| Hydroxypropylguar (JAGUAR HP 105 de RHODIA CHIMIE) | 0,2 g |
| Polyquaternium-10 (JR400 de RHODIA CHIMIE) | 0,5 g |
| Starch hydroxypropyltrimonium chloride (SENSOMER Cl-50 d'ONDEO) | 0,62 gMA |
| Hydroxyéthylcellulose (CELLOSIZE POLYMER PCG-10 de UNION CARBIDE ) | 0,7g |
| Glycérine | 5 g |
| Propylène glycol | 2 g |
| Acide citrique | 0,5 g |
| Parfum | qs |
| Conservateurs | qs |
| Eau qsp | 100 g |

La composition a été appliquée pendant 1 à 5 minutes, sur les cheveux lavés et essorés. On a ensuite rincé et séché les cheveux.
Les cheveux mouillés sont alors lisses et souples et les cheveux séchés sont brillants, souples et individualisés.

### EXEMPLE 4

On a préparé la composition suivante d'après-shampooing à rincer :

| | |
|---|---|
| Chlorure de béhénylamidopropyl dihydroxypropyl diméthyl 2,3- ammonium (LEXQUAT AMG-BEO de INOLEX) | 0,42 gMA |
| Quaternium-80 en solution à 50% MA dans le propylène glycol (ABIL QUAT 3272 de GOLDSCHMIDT) | 0,25 gMA |
| Hydroxypropylguar (JAGUAR HP 105 de RHODIA CHIMIE) | 0,4 g |
| Polyquaternium-10 (JR400 de RHODIA CHIMIE) | 0,5 g |
| Polyquaternium-44 (LUVIQUAT CARE de BASF) | 0,57 gMA |
| Hydroxyéthylcellulose (CELLOSIZE POLYMER PCG-10 de UNION CARBIDE ) | 0,3 g |
| Glycérine | 5 g |
| Acide citrique | 0,2 g |
| Parfum | qs |
| Conservateurs | qs |
| Eau qsp | 100g |

La composition a été appliquée pendant 1 à 5 minutes sur les cheveux lavés et essorés pendant 1 à 5 minutes. On a ensuite rincé et séché les cheveux.
Les cheveux mouillés sont alors lisses et souples et les cheveux séchés sont brillants, souples et individualisés.

## Revendications

1. Composition cosmétique non lavante, comprenant, dans un milieu cosmétiquement acceptable, au moins une silicone à groupements ammonium quaternaire, au moins un tensioactif cationique, au moins deux polymères cationiques différents et au moins un polymère épaississant non ionique et non associatif.

2. Composition cosmétique selon la revendication 1, **caractérisée en ce que** ladite silicone à groupements ammonium quaternaire est choisie parmi les silicones correspondant à une des formules générales suivantes : 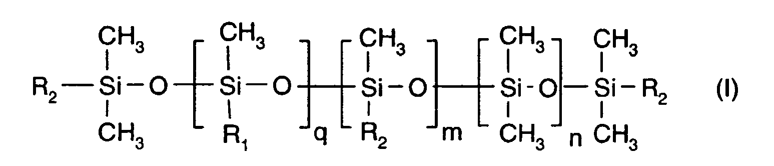 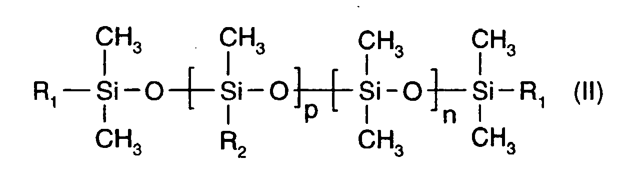 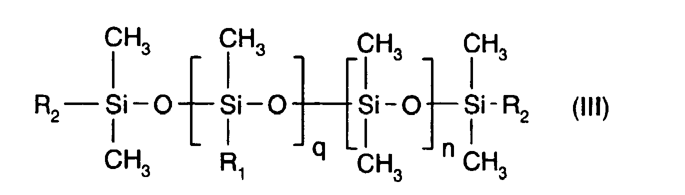 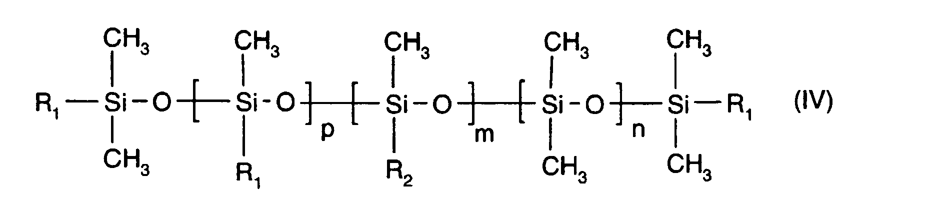 formules dans lesquelles :
- R₁, identique ou différent, représente un groupe alkyle, linéaire ou ramifié, en C₁-C₃₀, ou phényle ;
- R₂, identique ou différent, représente -C_{c}H_{2c}-O-(C₂H₄O)ₐ-(C₃H₆O)_{b}-R₅ ou -C_{c}H_{2c}-O-(C₄H₈O)ₐ-R₅ ;
R₅, identique ou différent, est choisi parmi les groupes de formule suivante : 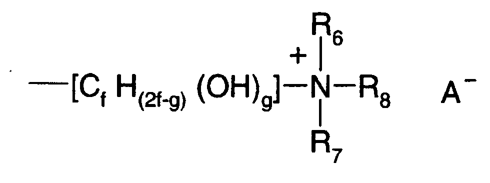
- les radicaux R₈ représentent indépendamment un radical alkyle en C₁₋₂₂ ou alcényle en C₂₋₂₂, linéaire ou ramifié, et portant éventuellement un ou plusieurs groupements OH, ou représentent un groupement CₕH₂ₕZCOR₉;
- R₆, R₇ et R₉, identiques ou différents, représentent des radicaux alkyle en C₁₋₂₂ ou alcényle en C₂₋₂₂, linéaires ou ramifiés, portant éventuellement un ou plusieurs groupements OH, ou R₇ peut former avec une partie de R₈ un hétérocycle (cycle avec au moins un hétéroatome tel que par exemple N, O, P) , l'hétéro cycle est notamment une imidazoline.
- m varie de 0 à 20 ;
- n varie de 0 à 500 ;
- p varie de 1 à 50 ;
- q varie de 0 à 20 ;
- a varie de 0 à 50 ;
- b varie de 0 à 50 ;
- c varie de 0 à 4 ;
- f varie de 0 à 4,
- g varie de 0 à 2, de préférence est égal à 1,
- h varie de 1 à 4, de préférence est égal à 3,
Z représente un atome d'oxygène ou NH,
A⁻ représente un anion minéral ou organique monovalent tel qu'un halogénure, un sulfate, ou un carboxylate.

3. Composition selon la revendication 2, **caractérisée en ce que** ladite silicone est de formule (III).

4. Composition selon la revendication 3, **caractérisée en ce que** ladite silicone répond à la formule générale (III) dans laquelle au moins l'une des conditions suivantes sont satisfaites :
- c est égal à 2 ou 3 ;
- R₁ désigne le groupe méthyle ;
- a et b sont égaux à zéro ;
- n varie de 0 à 100 ;
- q est égal à 0 ;
- f=3;
- g = 1 ;
- R₆ et R₇ désignent le groupe méthyle ;
- R₈ désigne le radical -(CH₂)-NHCOR₉.

5. Composition selon l'une quelconque des revendications 1 à 4, **caractérisée en ce que** ladite silicone est le Quaternium-80.

6. Composition cosmétique selon l'une quelconque des revendications 1 à 5, **caractérisée en ce que** les silicones à groupements ammonium quaternaire se présentent sous forme de solutions, de suspensions ou de dispersions dans l'eau.

7. Composition selon l'une quelconque des revendications 1 à 6, **caractérisée en ce qu**'elle contient la ou les silicones à groupements ammonium quaternaires à raison de 0,01% à 10 % en poids et, de préférence, de 0,1 à 5 % en poids de matière active par rapport au poids total de la composition.

8. Composition selon l'une quelconque des revendications précédentes, **caractérisée en ce que** les tensioactifs cationiques sont choisis parmi les sels d'amines grasses primaires, secondaires ou tertiaires, éventuellement polyoxyalkylénées, les sels d'ammonium quaternaire et leurs mélanges.

9. Composition selon la revendication 8, **caractérisée en ce que** les sels d'ammonium quaternaire sont choisis parmi :
- ceux qui présentent la formule générale (V) suivante : 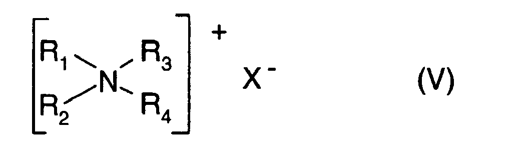
dans laquelle les symboles R₁ à R₄, qui peuvent être identiques ou différents, représentent un radical aliphatique, linéaire ou ramifié, comportant de 1 à 30 atomes de carbone, ou un radical aromatique tel que aryle ou alkylaryle ; X⁻ est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline ;
- les sels de diammonium quaternaire de formule (VII) : 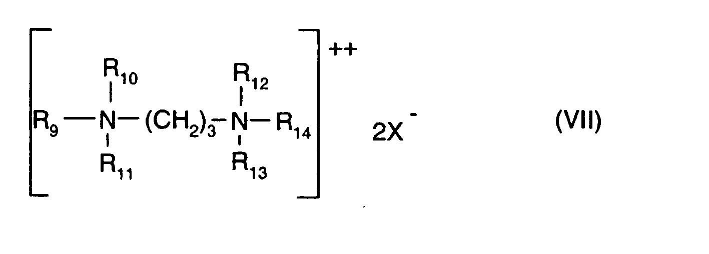
dans laquelle R₉ désigne un radical aliphatique comportant environ de 16 à 30 atomes de carbone, R₁₀, R₁₁, R₁₂, R₁₃ et R₁₄ , identiques ou différents sont choisis parmi l'hydrogène ou un radical alkyle comportant de 1 à 4 atomes de carbone, et X⁻ est un anion choisi dans le groupe des halogénures, acétates, phosphates, nitrates et méthylsulfates ;
- les sels d'ammonium quaternaire contenant au moins une fonction ester.

10. Composition selon la revendication 9, **caractérisée en ce que** les sels d'ammonium quaternaire de l'imidazoline sont choisis parmi ceux de formule (VI) suivante : 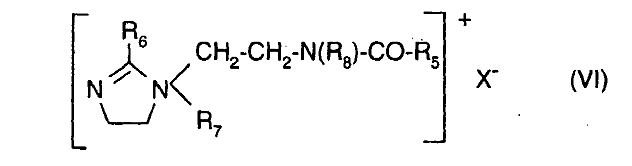 dans laquelle R₅ représente un radical alcényle ou alkyle comportant de 8 a 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène.

11. Composition selon l'une quelconque des revendications précédentes, **caractérisée en ce que** les tensioactifs cationiques sont choisis parmi le chlorure de béhényltriméthylammonium, le chlorure de cétyltriméthylammonium, le Quaternium-83, le chlorure de béhénylamidopropyl 2,3-dihydroxypropyl diméthyl ammonium et le chlorure de palmitylamidopropyltriméthylammonium.

12. Composition selon l'une quelconque des revendications précédentes, **caractérisée en ce que** les tensioactifs cationiques sont contenus en une quantité de 0,05 à 10 % en poids, de préférence de 0,1 à 5 % en poids par rapport au poids total de la composition.

13. Composition selon l'une quelconque des revendications 1 à 12, **caractérisée par le fait que** les polymères cationiques sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

14. Composition selon l'une quelconque des revendications précédentes, **caractérisée par le fait que** ledit polymère cationique est choisi parmi :
(1 ) les homopolymères ou copolymères dérivés d'esters ou d'amides acryliques ou méthacryliques et comportant au moins un des motifs de formules suivantes: 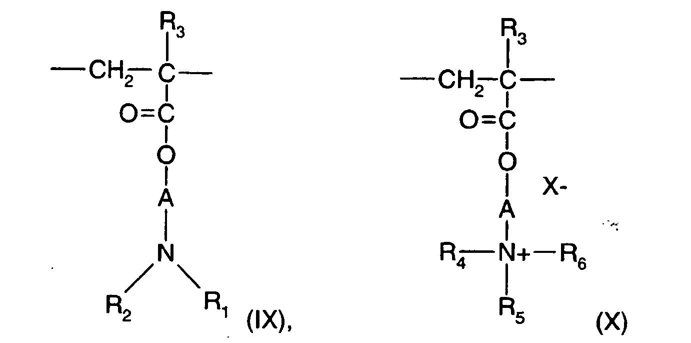 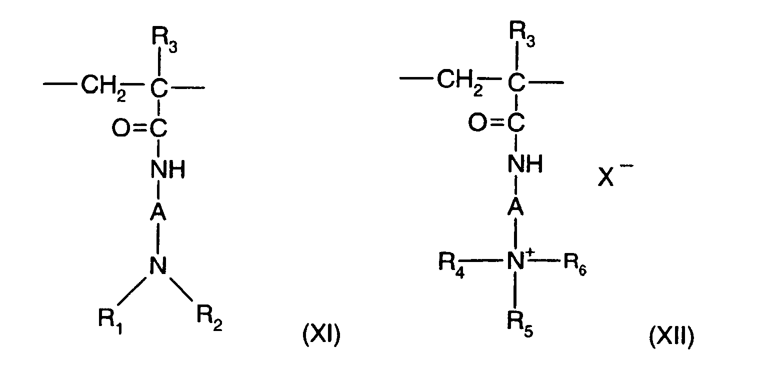 dans lesquelles:
R₃ , identiques ou différents, désignent un atome d'hydrogène ou un radical CH₃;
A, identiques ou différents, représentent un groupe alkyle, linéaire ou ramifié, de 1 à 6 atomes de carbone ou un groupe hydroxyalkyle de 1 à 4 atomes de carbone ;
R₄, R₅, R₆, identiques ou différents, représentent un groupe alkyle ayant de 1 à 18 atomes de carbone ou un radical benzyle;
R₁ et R₂ , identiques ou différents, représentent hydrogène ou un groupe alkyle ayant de 1 à 6 atomes de carbone;
X désigne un anion dérivé d'un acide minéral ou organique.
(2) Les polysaccharides cationiques,
(3)les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères,
(4)les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées.
(5)les polyaminoamides résultant de la condensation de polyalcoylènes polyamines avec des acides polycarboxyliques suivie d'une alcoylation par des agents bifonctionnels,
(6)les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone,
(7)les cyclopolymères d'alkyl diallyl amine ou de dialkyl diallyl ammonium
(8)les polymères de diammonium quaternaire contenant des motifs récurrents répondant à la formule : 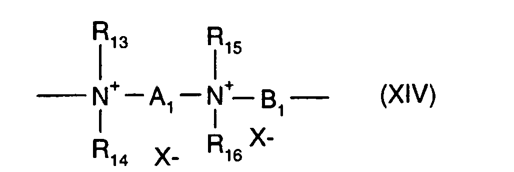 formule (XIV) dans laquelle :
R13, R14, R15 et R16, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R13, R14, R15 et R16, ensemble ou séparément, constituent avec les atomes d'azote auxquels ils sont rattachés des hétérocycles contenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
dans lequel D désigne :
a) un reste de glycol de formule : -O-Z-O-, où Z désigne un radical hydrocarboné linéaire ou ramifié ou un groupement répondant à l'une des formules suivantes :
-(CH2-CH2-O)x-CH2-CH2-
-[CH2-CH(CH3)-O]y-CH2-CH(CH3)-
où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent -CH2-CH2-S-S-CH2-CH2- ;
d) un groupement uréylène de formule : -NH-CO-NH- ;
De préférence, X⁻ est un anion minéral ou organique monovalent.
(9)les polymères de polyammonium quaternaires constitués de motifs de formule (XVI): 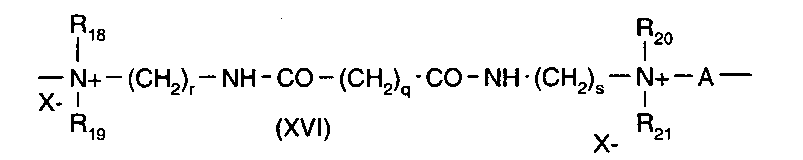 formule dans laquelle :
R18, R19, R20 et R21, identiques ou différents, représentent un atome d'hydrogène ou un radical méthyle, éthyle, propyle, β-hydroxyéthyle, β-hydroxypropyle ou -CH2CH2(OCH2CH2)pOH,
où p est égal à 0 ou à un nombre entier compris entre 1 et 6, sous réserve que R18, R19, R20 et R21 ne représentent pas simultanément un atome d'hydrogène,
r et s, identiques ou différents, sont des nombres entiers compris entre 1 et 6,
q est égal à 0 ou à un nombre entier compris entre 1 et 34,
X désigne un atome d'halogène,
A désigne un radical d'un dihalogénure ou représente de préférence -CH2-CH2-O-CH2-CH2-.
(10)Les polymères quaternaires de vinylpyrrolidone et de vinylimidazole,
(11) Les polyamines telles que « POLYETHYLENEGLYCOL (15) TALLOW POLYAMINE ».
(12) les polymères réticulés de sels de méthacryloyloxyalkyl(C₁-C₄) trialkyl(C₁₋C₄)ammonium,
(13) des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

15. Composition selon la revendication 14, **caractérisée par le fait que** lesdits polymères cationiques sont choisis parmi les cyclopolymères cationiques, les polysaccharides cationiques, les polymères quaternaires de vinylpyrrolidone et de vinylimidazole, et leurs mélanges.

16. Composition selon la revendication 15, **caractérisée par le fait que** ledit cyclopolymère est choisi parmi les homopolymères du chlorure de diallyldiméthylammonium et les copolymères du chlorure de diallyldiméthylammonium et d'acrylamide.

17. Composition selon la revendication 15, **caractérisée par le fait que** lesdits polysaccharides cationiques sont choisis parmi les amidons modifiés par un sel de 2,3-époxypropyl triméthylammonium, les gommes de guar modifiées par un sel de 2,3-époxypropyl triméthylammonium et les hydroxyéthylcelluloses ayant réagi avec un époxyde substitué par un groupement triméthylammonium.

18. Composition selon la revendication 15, **caractérisée par le fait que** lesdits polymères quaternaires de vinylpyrrolidone et de vinylimidazole sont choisis parmi les copolymères de vinylpyrrolidone et de sels de méthyl vinylimidazolium

19. Composition selon l'une quelconque des revendications précédentes, **caractérisée par le fait que** la composition comprend au moins un polysaccharide cationique et au moins un polymère quaternaire de vinylpyrrolidone et de vinylimidazole.

20. Composition selon l'une quelconque des revendications précédentes, **caractérisée par le fait que** la composition comprend au moins un homopolymère du chlorure de diallyldiméthylammonium et au moins un polymère quaternaire de vinylpyrrolidone et de vinylimidazole.

21. Composition selon l'une quelconque des revendications précédentes, **caractérisée en ce que** chaque polymère cationique est présent à une concentration allant de 0,001 % à 20 % en poids par rapport au poids total de la composition, de préférence de 0,01 % à 10 % en poids.

22. Composition selon l'une quelconque des revendications 1 à 21, **caractérisée en ce que** lesdits polymères épaississants non ioniques sont choisis parmi :
- les homopolymères et copolymères non-ioniques contenant des monomères à insaturation éthylénique de type ester et/ou amide ;
- les homo ou copolymères de vinylpyrrolidone,
- les polysaccharides.

23. Composition selon la revendication 22, **caractérisée en ce que** les homopolymères et copolymères non-ioniques contenant des monomères à insaturation éthylénique de type ester et/ou amide sont choisis parmi les polyacrylamides, les copolymères méthacrylate de méthyle / diméthacrylate d'éthylèneglycol), les copolymères méthacrylate de butyle / méthacrylate de méthyle, les polyméthacrylates de méthyle.

24. Composition selon la revendication 22, **caractérisée en ce que** les homo ou copolymères de vinylpyrrolidone sont notamment choisis parmi les homopolymères de vinylpyrrolidone réticulés.

25. Composition selon la revendication 22, **caractérisée par le fait que** les polysaccharides sont choisis parmi les glucanes, les amidons modifiés ou non, l'amylose, l'amylopectine, le glycogène, les dextranes, les celluloses et leurs dérivés (méthylcelluloses, hydroxyalkylcelluloses, éthylhydroxyéthylcelluloses), les mannanes, les xylanes, les lignines, les arabanes, les galactanes, les galacturonanes, la chitine, les chitosanes, les glucoronoxylanes, les arabinoxylanes, les xyloglucanes, les glucomannanes, les acides pectiques et les pectines, les arabinogalactanes, les carraghénines, les agars, les gommes arabiques, les gommes Tragacanthe, les gommes Ghatti, les gommes Karaya, les gommes de caroube, les galactomannanes telles que les gommes de guar et leurs dérivés non ioniques (hydroxypropyl guar), et leurs mélanges.

26. Composition selon l'une quelconque des revendications précédentes, **caractérisée en ce que** le polymère épaississant non ionique est présent à une concentration comprise entre 0,001 % et 20 % en poids par rapport au poids total de la composition, de préférence entre 0,01 % et 10 % en poids.

27. Composition selon l'une quelconque des revendications précédentes, **caractérisée en ce qu'**elle comprend en outre au moins un agent conditionneur additionnel.

28. Composition selon la revendication 27, **caractérisée en ce que** l'agent conditionneur est choisi parmi les silicones, les esters carboxyliques comprenant au moins 12 atomes de carbone, les huiles végétales, les huiles minérales, les huiles de synthèse et leurs mélanges.

29. Composition selon l'une quelconque des revendications précédentes, **caractérisée en ce que** le milieu aqueux cosmétiquement acceptable est constitué par de l'eau ou par un mélange d'eau et d'un solvant cosmétiquement acceptable.

30. Composition selon la revendication 29, **caractérisée en ce que** le solvant cosmétiquement acceptable est choisi parmi les alcools inférieur en C₁-C₄, les alkylèneglycols, les éthers de polyols, les alcanes en C₅-C₁₀, l'acétone, la méthyléthylcétone, les acétates d'alkyle en C₁-C₄, le diméthoxyéthane, le diéthoxyéthane, et leurs mélanges.

31. Composition selon l'une quelconque des revendications précédentes, **caractérisée en ce qu**'elle comprend en outre des additifs tels que des polymères anioniques, non ioniques ou amphotères, des épaississants non polymériques comme des acides ou des électrolytes, des opacifiants, des agents nacrants, des vitamines, des provitamines telles que le panthénol, des cires telles que les cires végétales, des céramides naturels ou synthétiques, des parfums, des colorants, des particules organiques ou minérales, des conservateurs, des agents de stabilisation du pH.

32. Composition selon l'une quelconque des revendications précédentes, **caractérisée par le fait qu**'elle se présente sous forme de shampooing, d'après-shampooing, de composition pour la permanente, le défrisage, la coloration ou la décoloration des cheveux, de composition à rincer à appliquer entre les deux étapes d'une permanente ou d'un défrisage.

33. Composition selon l'une quelconque des revendications précédentes, **caractérisée en ce qu**'elle est se présente sous la forme d'un après-shampoing à rincer.

34. Utilisation d'une composition telle que définie dans l'une quelconque des revendications précédentes pour le conditionnement ou pour le soin des matières kératiniques.

35. Procédé de traitement des matières kératiniques, telles que les cheveux, **caractérisé en ce qu**'il consiste à appliquer sur lesdites matières une quantité efficace d'une composition cosmétique selon l'une des revendications 1 à 33, puis à effectuer éventuellement un rinçage.

36. Utilisation d'une composition telle que définie dans l'une quelconque des revendications 1 à 33 pour apporter de la brillance aux cheveux.

37. Utilisation d'une composition telle que définie dans l'une quelconque des revendications 1 à 33 pour apporter de la souplesse aux cheveux.

## Claims

1. Non-washing cosmetic composition comprising, in a cosmetically acceptable medium, at least one silicone containing quaternary ammonium groups, at least one cationic surfactant, at least two different cationic polymers and at least one non-ionic and non-associative thickening polymer.

2. Cosmetic composition according to Claim 1, **characterized in that** said silicone containing quaternary ammonium groups is chosen from the silicones corresponding to one of the following general formulae: 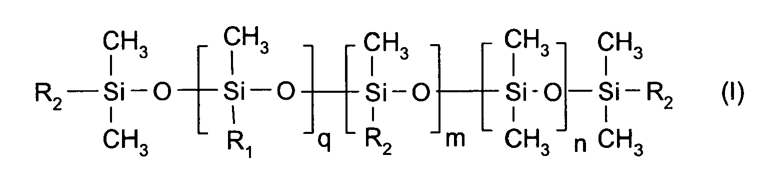 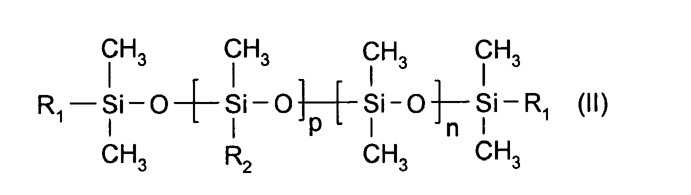 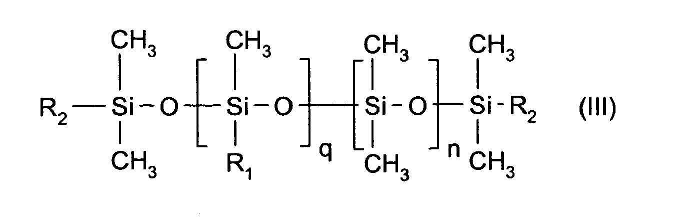 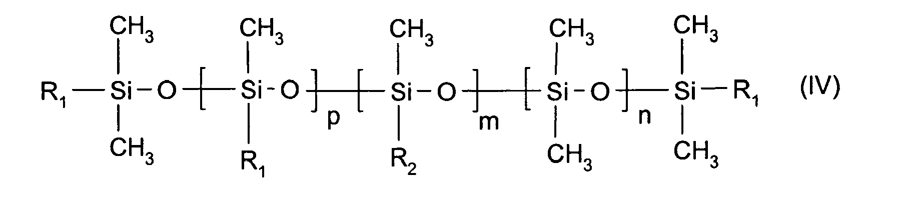 in which formulae:
- R₁, which may be identical or different, represents a C₁-C₃₀ linear or branched alkyl group, or phenyl;
- R₂, which may be identical or different, represents
- C_{c}H_{2c}-O- (C₂H₄O)ₐ-(C₃H₆O)_{b}-R₅ or -C_{c}H_{2c}-O- (C₄H₈O)ₐ-R₅;
R₅, which may be identical or different, is chosen from the groups having the following formula: 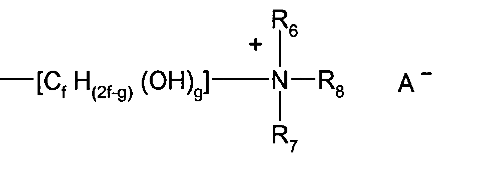
- the radicals R₈ independently represent a linear or branched C₁₋₂₂ alkyl or C₂₋₂₂ alkenyl radical optionally bearing one or more OH groups, or represent a group CₕH₂ₕZCOR₉;
- R₆, R₇ and R₉, which may be identical or different, represent linear or branched C₁₋₂₂ alkyl or C₂₋₂₂ alkenyl radicals optionally bearing one or more OH groups, or R₇ may form with a portion of R₈ a heterocycle (ring with at least one hetero atom, for instance N, O or P), the heterocycle is especially an imidazoline;
- m ranges from 0 to 20;
- n ranges from 0 to 500;
- p ranges from 1 to 50;
- q ranges from 0 to 20;
- a ranges from 0 to 50;
- b ranges from 0 to 50;
- c ranges from 0 to 4;
- f ranges from 0 to 4 ;
- g ranges from 0 to 2 and is preferably equal to 1;
- h ranges from 1 to 4 and is preferably equal to 3;
Z represents an oxygen atom or NH,
A⁻ represents a monovalent mineral or organic anion such as a halide, a sulfate or a carboxylate.

3. Composition according to Claim 2, **characterized in that** said silicone is of formula (III).

4. Composition according to Claim 3, **characterized in that** said silicone corresponds to the general formula (III) in which at least one of the following conditions is met:
- c is equal to 2 or 3;
- R₁ denotes a methyl group;
- a and b are equal to zero;
- n ranges from 0 to 100;
- q is equal to 0;
- f = 3;
- g = 1;
- R₆ and R₇ denote a methyl group;
- R₈ denotes a radical -(CH₂)-NHCOR₉.

5. Composition according to any one of Claims 1 to 4, **characterized in that** said silicone is Quaternium-80.

6. Cosmetic composition according to any one of Claims 1 to 5, **characterized in that** the silicones containing quaternary ammonium groups are present in the form of solutions, suspensions or dispersions in water.

7. Composition according to any one of Claims 1 to 6, **characterized in that** it contains the silicone(s) containing quaternary ammonium groups in a proportion of from 0.01% to 10% by weight and preferably from 0.1% to 5% by weight of active material relative to the total weight of the composition.

8. Composition according to any one of the preceding claims, **characterized in that** the cationic surfactants are chosen from optionally polyoxyalkylenated primary, secondary or tertiary fatty amine salts, and quaternary ammonium salts, and mixtures thereof.

9. Composition according to Claim 8, **characterized in that** the quaternary ammonium salts are chosen from:
- those of general formula (V) below: 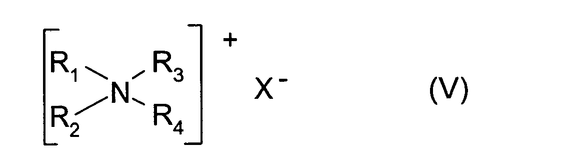
in which the symbols R₁ to R₄, which may be identical or different, represent a linear or branched aliphatic radical containing from 1 to 30 carbon atoms, or an aromatic radical such as aryl or alkylaryl; X- is an anion chosen from the group of halides, phosphates, acetates, lactates, (C₂-C₆) alkyl sulfates and alkyl- or alkylaryl-sulfonates;
- quaternary ammonium salts of imidazoline;
- diquaternary ammonium salts of formula (VII): 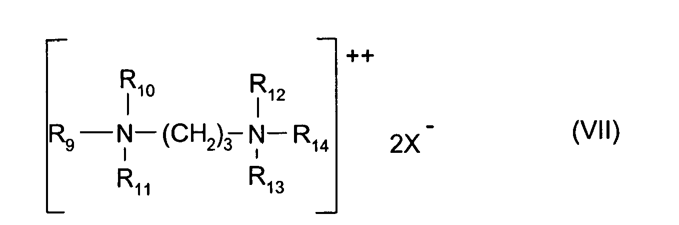
in which R₉ denotes an aliphatic radical containing from about 16 to 30 carbon atoms, R₁₀, R₁₁, R₁₂, R₁₃ and R₁₄, which may be identical or different, are chosen from hydrogen and an alkyl radical containing from 1 to 4 carbon atoms, and X⁻ is an anion chosen from the group of halides, acetates, phosphates, nitrates and methyl sulfates;
- quaternary ammonium salts containing at least one ester function.

10. Composition according to Claim 9, **characterized in that** the quaternary ammonium salts of imidazoline are chosen from those of formula (VI) below: 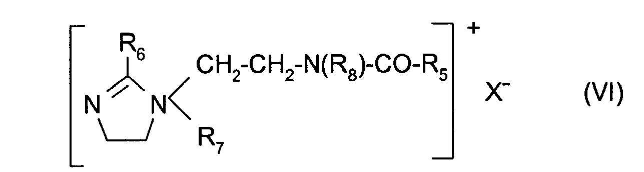 in which R₅ represents an alkenyl or alkyl radical containing from 8 to 30 carbon atoms, for example fatty acid derivatives of tallow, R₆ represents a hydrogen atom, a C₁-C₄ alkyl radical or an alkenyl or alkyl radical containing from 8 to 30 carbon atoms, R₇ represents a C₁-C₄ alkyl radical, R₈ represents a hydrogen atom or a C₁-C₄ alkyl radical, and X is an anion chosen from the group of halides, phosphates, acetates, lactates, alkyl sulfates, alkylsulfonates or alkylarylsulfonates. R₅ and R₆ preferably denote a mixture of alkenyl or alkyl radicals containing from 12 to 21 carbon atoms, such as, for example, fatty acid derivatives of tallow, R₇ denotes methyl and R₈ denotes hydrogen.

11. Composition according to any one of the preceding claims, **characterized in that** the cationic surfactants are chosen from behenyltrimethylammonium chloride, cetyltrimethylammonium chloride, Quaternium-83, behenylamidopropyl-2,3-dihydroxypropyldimethylammonium chloride and palmitylamidopropyltrimethylammonium chloride.

12. Composition according to any one of the preceding claims, **characterized in that** the cationic surfactants are in a content of from 0.05% to 10% by weight and preferably from 0.1% to 5% by weight relative to the total weight of the composition.

13. Composition according to any one of Claims 1 to 12, **characterized in that** the cationic polymers are chosen from those containing units comprising primary, secondary, tertiary and/or quaternary amine groups that may either form part of the main polymer chain or may be borne by a side substituent directly attached thereto.

14. Composition according to any one of the preceding claims, **characterized in that** said cationic polymer is chosen from:
(1) homopolymers or copolymers derived from acrylic or methacrylic esters or amides and comprising at least one of the units of the following formulae: 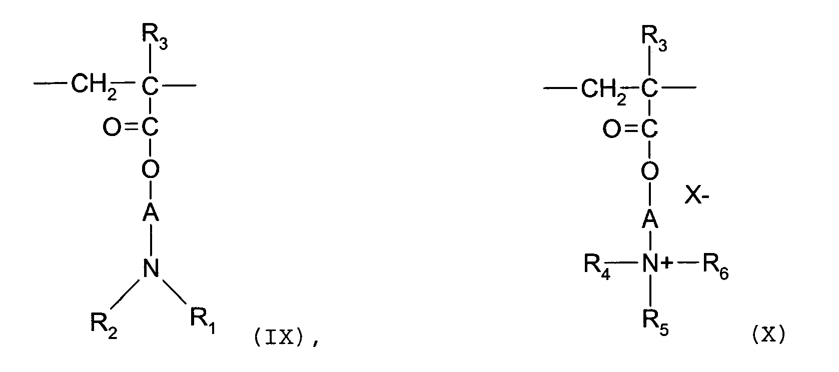 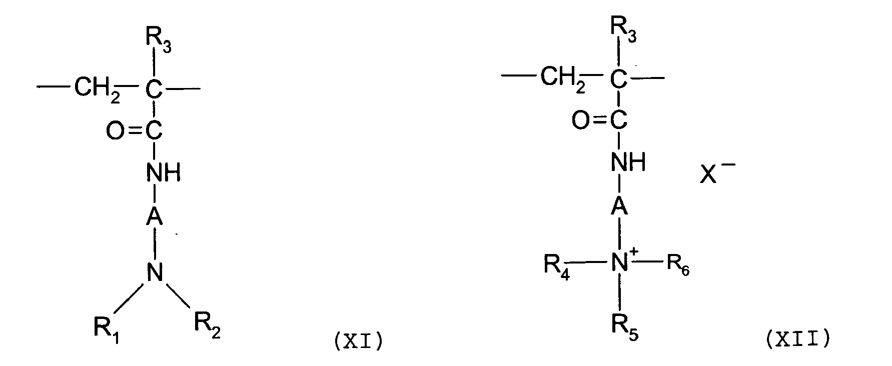 in which:
R₃, which may be identical or different, denote a hydrogen atom or a CH₃ radical;
A, which may be identical or different, represent a linear or branched alkyl group of 1 to 6 carbon atoms or a hydroxyalkyl group of 1 to 4 carbon atoms;
R₄, R₅ and R₆, which may be identical or different, represent an alkyl group containing from 1 to 18 carbon atoms or a benzyl radical;
R₁ and R₂, which may be identical or different, represent hydrogen or an alkyl group containing from 1 to 6 carbon atoms;
X denotes an anion derived from a mineral or organic acid,
(2) cationic polysaccharides,
(3) polymers consisting of piperazinyl units and of divalent alkylene or hydroxyalkylene radicals containing straight or branched chains, optionally interrupted by oxygen, sulfur or nitrogen atoms or by aromatic or heterocyclic rings, as well as the oxidation and/or quaternization products of these polymers,
(4) water-soluble polyaminoamides prepared in particular by polycondensation of an acidic compound with a polyamine; these polyaminoamides can be crosslinked with an epihalohydrin, a diepoxide, a dianhydride, an unsaturated dianhydride, a bisunsaturated derivative, a bishalohydrin, a bisazetidinium, a bishaloacyldiamine, a bisalkyl halide or alternatively with an oligomer resulting from the reaction of a difunctional compound which is reactive with a bishalohydrin, a bisazetidinium, a bishaloacyldiamine, a bisalkyl halide, an epilhalohydrin, a diepoxide or a bisunsaturated derivative; the crosslinking agent being used in proportions ranging from 0.025 to 0.35 mol per amine group of the polyaminoamide; these polyaminoamides can be alkylated or, if they contain one or more tertiary amine functions, they can be quaternized,
(5) polyaminoamides resulting from the condensation of polyalkylene polyamines with polycarboxylic acids followed by alkylation with difunctional agents,
(6) polymers obtained by reaction of a polyalkylene polyamine containing two primary amine groups and at least one secondary amine group with a dicarboxylic acid chosen from diglycolic acid and saturated aliphatic dicarboxylic acids having from 3 to 8 carbon atoms,
(7) cyclopolymers of alkyldiallylamine or of dialkyldiallylammonium,
(8) quaternary diammonium polymers containing repeating units corresponding to the formula: 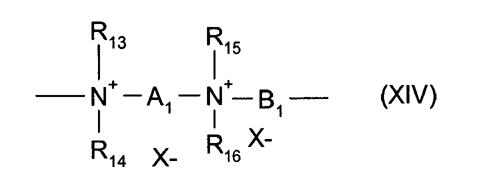 in which formula (XIV) :
R₁₃, R₁₄, R₁₅ and R₁₆, which may be identical or different, represent aliphatic, alicyclic or arylaliphatic radicals containing from 1 to 20 carbon atoms or lower hydroxyalkylaliphatic radicals, or alternatively R₁₃, R₁₄, R₁₅ and R₁₆, together or separately, constitute, with the nitrogen atoms to which they are attached, heterocycles optionally containing a second hetero atom other than nitrogen, or alternatively R₁₃, R₁₄, R₁₅ and R₁₆ represent a linear or branched C₁-C₆ alkyl radical substituted with a nitrile, ester, acyl or amide group or a group -CO-O-R₁₇-D or -CO-NH-R₁₇-D where R₁₇ is an alkylene and D is a quaternary ammonium group;
A₁ and B₁ represent polymethylene groups containing from 2 to 20 carbon atoms, which groups may be linear or branched, saturated or unsaturated, and which may contain, linked to or intercalated in the main chain, one or more aromatic rings or one or more oxygen or sulfur atoms or sulfoxide, sulfone, disulfide, amino, alkylamino, hydroxyl, quaternary ammonium, ureido, amide or ester groups, and
X⁻ denotes an anion derived from a mineral or organic acid;
A₁, R₁₃ and R₁₅ can form, with the two nitrogen atoms to which they are attached, a piperazine ring; in addition, if A₁ denotes a linear or branched, saturated or unsaturated alkylene or hydroxyalkylene radical, B₁ can also denote a group (CH₂)ₙ-CO-D-OC-(CH₂)ₙ-
in which D denotes:
a) a glycol residue of formula: -O-Z-O-, where Z denotes a linear or branched hydrocarbon-based radical or a group corresponding to one of the following formulae:
-(CH₂-CH₂-O)ₓ-CH₂-CH₂-
-[CH₂-CH(CH₃)-O]_{y}-CH₂-CH(CH₃)-
where x and y denote an integer from 1 to 4, representing a defined and unique degree of polymerization or any number from 1 to 4 representing an average degree of polymerization;
b) a bissecondary diamine residue such as a piperazine derivative;
c) a bisprimary diamine residue of formula: -NH-Y-NH-, where Y denotes a linear or branched hydrocarbon-based radical, or alternatively the divalent radical
-CH₂-CH₂-S-S-CH₂-CH₂-;
d) a ureylene group of formula: -NH-CO-NH-;
preferably, X⁻ is a monovalent mineral or organic anion,
(9) polyquaternary ammonium polymers consisting of units of formula (XVI): 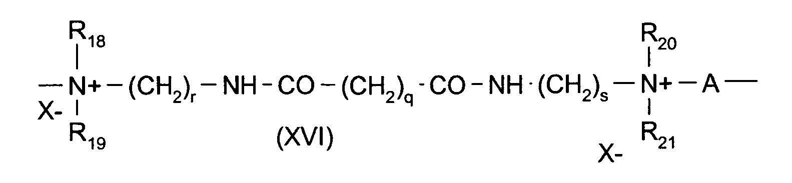 in which formula:
R₁₈, R₁₉, R₂₀ and R₂₁, which may be identical or different, represent a hydrogen atom or a methyl, ethyl, propyl, β-hydroxyethyl, β-hydroxypropyl or -CH₂CH₂(OCH₂CH₂)ₚOH radical,
where p is equal to 0 or to an integer between 1 and 6, with the proviso that R₁₈, R₁₉, R₂₀ and R₂₁ do not simultaneously represent a hydrogen atom,
r and s, which may be identical or different, are integers between 1 and 6,
q is equal to 0 or to an integer between 1 and 34,
X denotes a halogen atom,
A denotes a dihalide radical or preferably represents -CH₂-CH₂-O-CH₂-CH₂-,
(10) quaternary polymers of vinylpyrrolidone and of vinylimidazole,
(11) polyamines such as "Polyethylene Glycol (15) Tallow Polyamine",
(12) crosslinked methacryloyloxy(C₁-C₄)alkyltri(C₁-C₄)-alkylammonium salt polymers,
(13) polyalkyleneimines, in particular polyethylene-imines, polymers containing vinylpyridine or vinyl-pyridinium units, condensates of polyamines and of epichlorohydrin, polyquaternary ureylenes and chitin derivatives.

15. Composition according to Claim 14, **characterized in that** said cationic polymers are chosen from cationic cyclopolymers, cationic polysaccharides and quaternary polymers of vinylpyrrolidone and of vinylimidazole, and mixtures thereof.

16. Composition according to Claim 15, **characterized in that** said cyclopolymer is chosen from diallyldimethylammonium chloride homopolymers and copolymers of diallyldimethylammonium chloride and of acrylamide.

17. Composition according to Claim 15, **characterized in that** said cationic polysaccharides are chosen from starches modified with a 2,3-epoxypropyltrimethylammonium salt, guar gums modified with a 2,3-epoxypropyltrimethylammonium salt and hydroxyethyl-celluloses that have reacted with an epoxide substituted with a trimethylammonium group.

18. Composition according to Claim 15, **characterized in that** said quaternary polymers of vinylpyrrolidone and of vinylimidazole are chosen from copolymers of vinylpyrrolidone and of methylvinylimidazolium salts.

19. Composition according to any one of the preceding claims, **characterized in that** the composition comprises at least one cationic polysaccharide and at least one quaternary polymer of vinylpyrrolidone and of vinylimidazole.

20. Composition according to any one of the preceding claims, **characterized in that** the composition comprises at least one diallyldimethylammonium chloride homopolymer and at least one quaternary polymer of vinylpyrrolidone and of vinylimidazole.

21. Composition according to any one of the preceding claims, **characterized in that** each cationic polymer is present in a concentration ranging from 0.001% to 20% by weight and preferably from 0.01% to 10% by weight relative to the total weight of the composition.

22. Composition according to any one of Claims 1 to 21, **characterized in that** said non-ionic thickening polymers are chosen from:
- non-ionic homopolymers and copolymers containing ethylenically unsaturated monomers of ester and/or amide type;
- vinylpyrrolidone homopolymers or copolymers;
- polysaccharides.

23. Composition according to Claim 22, **characterized in that** the non-ionic homopolymers and copolymers containing ethylenically unsaturated monomers of ester and/or amide type are chosen from polyacrylamides, methyl methacrylate/ethylene glycol dimethacrylate copolymers, butyl methacrylate/ methyl methacrylate copolymers and polymethyl methacrylates.

24. Composition according to Claim 22, **characterized in that** the vinylpyrrolidone homopolymers or copolymers are chosen especially from crosslinked vinylpyrrolidone homopolymers.

25. Composition according to Claim 22, **characterized in that** the polysaccharides are chosen from glucans, modified or unmodified starches, amylose, amylopectin, glycogen, dextrans, celluloses and derivatives thereof (methylcelluloses, hydroxyalkylcelluloses and ethylhydroxyethylcelluloses), mannans, xylans, lignins, arabans, galactans, galacturonans, chitin, chitosans, glucuronoxylans, arabinoxylans, xyloglucans, glucomannans, pectic acids and pectins, arabinogalactans, carrageenans, agars, gum arabics, gum tragacanths, ghatti gums, karaya gums, carob gums, galactomannans such as guar gums and non-ionic derivatives thereof (hydroxypropyl guar), and mixtures thereof.

26. Composition according to any one of the preceding claims, **characterized in that** the non-ionic thickening polymer is present in a concentration of between 0.001% and 20% by weight, and preferably between 0.01% and 10% by weight, relative to the total weight of the composition.

27. Composition according to any one of the preceding claims, **characterized in that** it also comprises at least one additional conditioner.

28. Composition according to Claim 27, **characterized in that** the conditioner is chosen from silicones, carboxylic esters containing at least 12 carbon atoms, plant oils, mineral oils and synthetic oils, and mixtures thereof.

29. Composition according to any one of the preceding claims, **characterized in that** the cosmetically acceptable aqueous medium consists of water or of a mixture of water and a cosmetically acceptable solvent.

30. Composition according to Claim 29, **characterized in that** the cosmetically acceptable solvent is chosen from C₁-C₄ lower alcohols, alkylene glycols, polyol ethers, C₅-C₁₀ alkanes, acetone, methyl ethyl ketone, C₁-C₄ alkyl acetates, dimethoxyethane and diethoxyethane, and mixtures thereof.

31. Composition according to any one of the preceding claims, **characterized in that** it also comprises additives such as anionic, non-ionic or amphoteric polymers, non-polymeric thickeners, for instance acids or electrolytes, opacifiers, nacreous agents, vitamins, provitamins such as panthenol, waxes such as plant waxes, natural or synthetic ceramides, fragrances, colorants, organic or mineral particles, preserving agents and pH stabilizers.

32. Composition according to any one of the preceding claims, **characterized in that** it is in the form of a shampoo, a conditioner, a permanent-waving, relaxing, dyeing or bleaching composition for the hair or a rinse-out composition to be applied between the two steps of a permanent-waving or relaxing operation.

33. Composition according to any one of the preceding claims, **characterized in that** it is in the form of a rinse-out conditioner.

34. Use of a composition as defined in any one of the preceding claims, for conditioning or caring for keratin materials.

35. Process for treating keratin materials, such as the hair, **characterized in that** it consists in applying to said materials an effective amount of a cosmetic composition according to one of Claims 1 to 33, optionally followed by rinsing the keratin materials.

36. Use of a composition as defined in any one of Claims 1 to 33, for giving the hair sheen.

37. Use of a composition as defined in any one of Claims 1 to 33, for giving the hair suppleness.

## Patentansprüche

1. Nichtreinigende kosmetische Zusammensetzung, die in einem kosmetisch akzeptablen Medium mindestens ein Silicon mit quartären Ammoniumgruppen, mindestens einen kationischen grenzflächenaktiven Stoff, mindestens zwei unterschiedliche kationische Polymere und mindestens ein nichtionisches und nicht assoziatives, verdickendes Polymer enthält.

2. Kosmetische Zusammensetzung nach Anspruch, **dadurch gekennzeichnet, dass** das Silicon mit quartären Ammoniumgruppen unter den Siliconen ausgewählt ist, die einer der folgenden allgemeinen Formeln entsprechen: 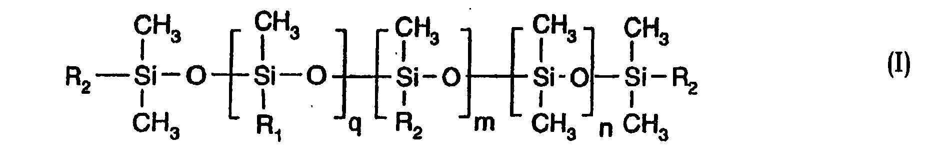 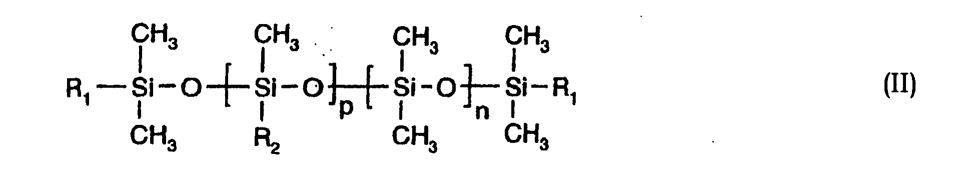 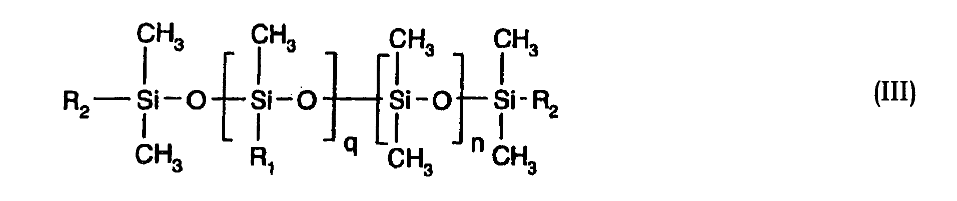 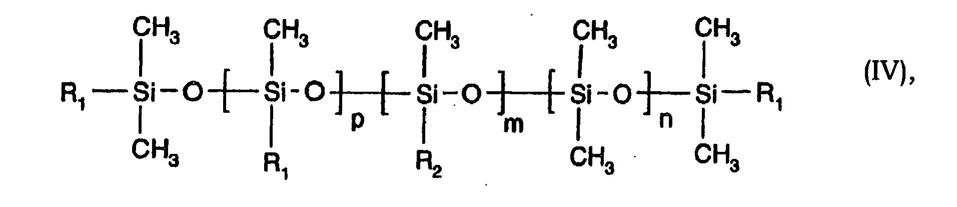 wobei in den Formeln bedeuten:
- die Gruppen R₁, die gleich oder verschieden sind, eine geradkettige oder verzweigte C₁₋₃₀-Alkylgruppe oder eine Phenylgruppe;
- die Gruppen R₂, die gleich oder verschieden sind, -C_{c}H_{2c}-O-(C₂H₄O)ₐ-(C₃H₆O)_{b}-R₅ oder -C_{c}H_{2c}-O-(C₄H₈O)ₐ-R₅; die Gruppen R₅, die gleich oder verschieden sind, sind unter den Gruppen der folgenden Formel ausgewählt: 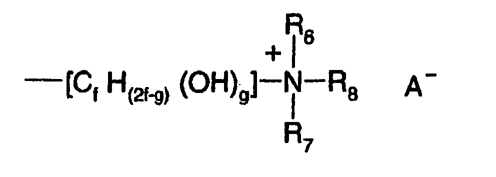
- die Gruppen R₈ bedeuten unabhängig voneinander eine geradkettige oder verzweigte C₁₋₂₂-Alkyl- oder C₂₋₂₂-Alkenylgruppe, wobei sie gegebenenfalls eine oder mehrere OH-Gruppen enthalten können, oder sie bedeuten eine Gruppe CₕH₂ₕZCOR₉;
- die Gruppen R₆, R₇ und R₉, die gleich oder verschieden sind, bedeuten geradkettige oder verzweigte C₁₋₂₂-Alkyl- oder C₂₋₂₂-Alkenylgruppen, wobei sie gegebenenfalls eine oder mehrere OH-Gruppen enthalten können, oder R₇ kann mit einem Teil von R₈ einen Heterocyclus bilden (Ring mit mindestens einem Heteroatom, wie beispielsweise N, O, P), wobei es sich bei dem Heterocyclus insbesondere um ein Imidazolin handelt;
- m liegt im Bereich von 0 bis 20;
- n liegt im Bereich von 0 bis 500;
- p liegt im Bereich von 1 bis 50;
- q liegt im Bereich von 0 bis 20;
- a liegt im Bereich von 0 bis 50;
- b liegt im Bereich von 0 bis 50;
- c liegt im Bereich von 0 bis 4;
- f liegt im Bereich von 0 bis 4;
- g liegt im Bereich von 0 bis 2 und beträgt vorzugsweise 1;
- h liegt im Bereich von 1 bis 4 und bedeutet vorzugsweise 3;
- Z bedeutet ein Sauerstoffatom oder NH;
- A⁻ ein einwertiges anorganisches oder organisches Anion, beispielsweise ein Halogenid, Sulfat oder Carboxylat.

3. Zusammensetzung nach Anspruch 2, **dadurch gekennzeichnet, dass** das Silicon die Formel (III) aufweist.

4. Zusammensetzung nach Anspruch 3, **dadurch gekennzeichnet, dass** das Silicon der allgemeinen Formel (III) entspricht, worin mindestens eine der folgenden Bedingungen erfüllt ist:
- c beträgt 2 oder 3;
- R₁ bedeutet Methyl;
- a und b bedeuten beide 0;
- n liegt im Bereich von 0 bis 100;
- q beträgt 0;
- f = 3,
- g = 1;
- R₆ und R₇ bedeuten Methyl;
- R₈ ist die Gruppe -(CH₂)-NHCOR₉.

5. Zusammensetzung nach einem der Ansprüche 1 bis 4, **dadurch gekennzeichnet, dass** das Silicon das Quaternium-80 ist.

6. Kosmetische Zusammensetzung nach einem der Ansprüche 1 bis 5, **dadurch gekennzeichnet, dass** die Silicone mit quartären Ammoniumgruppen in Form von Lösungen, Suspensionen oder Dispersionen in Wasser vorliegen.

7. Zusammensetzung nach einem der Ansprüche 1 bis 6, **dadurch gekennzeichnet, dass** sie das oder die Silicon(e) mit quartären Ammoniumgruppen in einer Menge von 0,01 bis 10 Gew.-% und vorzugsweise 0,1 bis 5 Gew.-% wirksame Substanz, bezogen auf das Gesamtgewicht der Zusammensetzung, enthält.

8. Zusammensetzung nach einem der vorhergehenden Ansprüche, **dadurch gekennzeichnet, dass** die kationischen grenzflächenaktiven Stoffe unter den Salzen von primären, sekundären oder tertiären Aminen, die gegebenenfalls polyalkoxyliert sind, quartären Ammoniumsalzen und deren Gemischen ausgewählt sind.

9. Zusammensetzung nach Anspruch 8, **dadurch gekennzeichnet, dass** die quartären Ammoniumsalze ausgewählt sind unter:
- solchen, die die folgende allgemeine Formel (V) aufweisen: 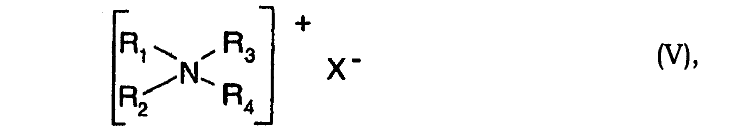
worin die Symbole R₁ bis R₄, die gleich oder verschieden sein können, eine aliphatische, geradkettige oder verzweigte Gruppe mit 1 bis 30 Kohlenstoffatomen oder eine aromatische Gruppe, wie Aryl oder Alkylaryl bedeuten; X⁻ ein Anion ist, das unter den Halogeniden, Phosphaten, Acetaten, Lactaten, Alkyl(C₂₋₆)sulfaten, Alkyl- oder Alkylarylsulfonaten ausgewählt ist;
- quartären Ammoniumsalzen von Imidazolin;
- quartären Diammoniumsalzen der Formel (VII): 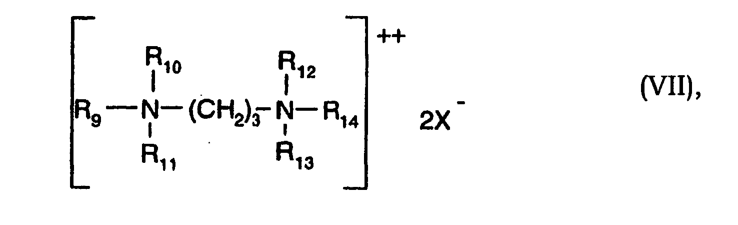
worin R₉ eine aliphatische Gruppe bedeutet, die etwa 16 bis 30 Kohlenstoffatome aufweist, R₁₀, R₁₁, R₁₂, R₁₃ und R₁₄, die gleich oder verschieden sind, unter Wasserstoff oder einer Alkylgruppe mit 1 bis 4 Kohlenstoffatomen ausgewählt sind, und X⁻ ein Anion ist, das unter den Halogeniden, Acetaten, Phosphaten, Nitraten und Methylsulfaten ausgewählt ist;
- quartären Ammoniumsalzen, die mindestens eine Esterfunktion aufweisen.

10. Zusammensetzung nach Anspruch 9, **dadurch gekennzeichnet, dass** die quartären Ammoniumsalze von Imidazolin unter den Verbindungen der folgenden Formel (VI) ausgewählt sind: 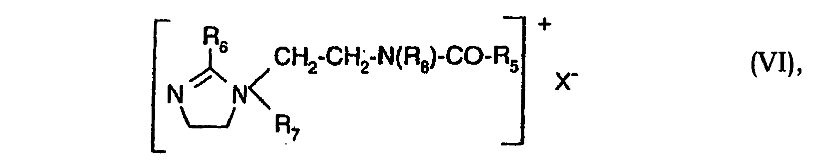 worin R₅ eine Alkenyl- oder Alkylgruppe bedeutet, die 8 bis 30 Kohlenstoffatome aufweist und beispielsweise von Talgfettsäuren abgeleitet ist, R₆ ein Wasserstoffatom, C₁₋₄-Alkyl oder eine Alkenyl- oder Alkylgruppe mit 8 bis 30 Kohlenstoffatomen bedeutet, R₇ C₁₋₄-Alkyl bedeutet, R₈ ein Wasserstoffatom oder C₁₋₄-Alkyl ist und X ein Anion ist, das unter den Halogeniden, Phosphaten, Acetaten, Lactaten, Alkylsulfaten, Alkyl- oder Alkylarylsulfonaten ausgewählt ist; vorzugsweise bedeuten die Gruppen R₅ und R₆ ein Gemisch aus Alkenyl- oder Alkylgruppen mit 12 bis 21 Kohlenstoffatomen, die beispielsweise von Talgfettsäuren abgeleitet sind, R₇ ist Methyl und R₈ bedeutet Wasserstoff.

11. Zusammensetzung nach einem der vorhergehenden Ansprüche, **dadurch gekennzeichnet, dass** die kationischen grenzflächenaktiven Stoffe unter Behenyltrimethylammoniumchlorid, Cetyltrimethylammoniumchlorid, Quaternium-83, Behenylamidopropyl-2,3-dihydroxypropyldimethylammoniumchlorid und Palmitylamidopropyltrimethylammoniumchlorid ausgewählt sind.

12. Zusammensetzung nach einem der vorhergehenden Ansprüche, **dadurch gekennzeichnet, dass** die kationischen grenzflächenaktiven Stoffe in einer Menge von 0,05 bis 10 Gew.-% und vorzugsweise 0,1 bis 5 Gew.-%, bezogen auf das Gesamtgewicht der Zusammensetzung, enthalten sind.

13. Zusammensetzung nach einem der Ansprüche 1 bis 12, **dadurch gekennzeichnet, dass** die kationischen Polymere unter den Polymeren ausgewählt sind, die Einheiten enthalten, die entweder als Teil der Polymerhauptkette oder an einem direkt an die Polymerhauptkette gebunden seitlichen Substituenten Einheiten mit primären, sekundären, tertiären und/oder quartären Aminogruppen enthalten.

14. Zusammensetzung nach einem der vorhergehenden Ansprüche, **dadurch gekennzeichnet, dass** das kationische Polymer ausgewählt ist unter:
(1) Homopolymeren oder Copolymeren, die von Acrylsäureestern, Methacrylsäureestern, Acrylamiden oder Methacrylamiden abgeleitet sind und mindestens eine Einheit der folgenden Formel enthalten: 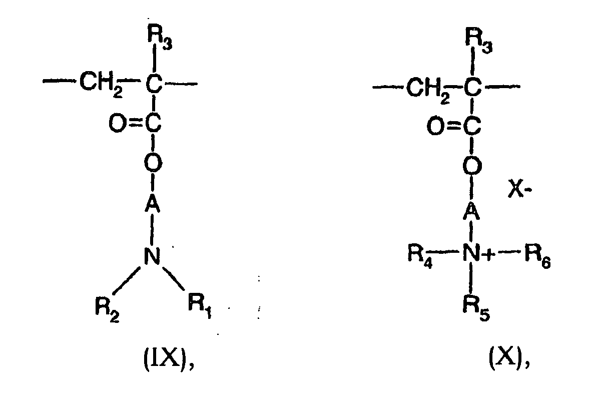 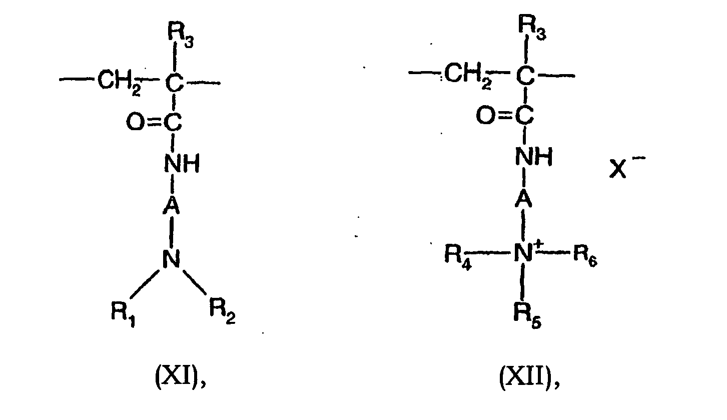 worin bedeuten:
die Gruppen R₃, die gleich oder verschieden sind, ein Wasserstoffatom oder CH₃,
die Gruppen A, die gleich oder verschieden sind, eine geradkettige oder verzweigte Alkylgruppe mit 1 bis 6 Kohlenstoffatomen oder eine Hydroxyalkylgruppe mit 1 bis 4 Kohlenstoffatomen;
die Gruppen R₄, R₅ und R₆, die gleich oder verschieden sind, eine Alkylgruppe mit 1 bis 18 Kohlenstoffatomen oder eine Benzylgruppe;
die Gruppen R₁ und R₂, die gleich oder verschieden sind, Wasserstoff oder eine Alkylgruppe mit 1 bis 6 Kohlenstoffatomen;
X ein Anion, das von einer anorganischen oder organischen Säure abgeleitet ist;
(2) kationischen Polysacchariden;
(3) Polymeren, die aus Piperazinyleinheiten und zweiwertigen Alkylen- oder Hydroxyalkylengruppen mit geraden oder verzweigten Ketten bestehen, die gegebenenfalls durch Sauerstoffatome, Schwefelatome, Stickstoffatome oder aromatische oder heterocyclische Ringe unterbrochen sind, sowie den Oxidationsprodukten und/oder Quaternisierungsprodukten dieser Polymere;
(4) wasserlöslichen Polyaminoamiden, die insbesondere durch Polykondensation einer sauren Verbindung mit einem Polyamin hergestellt sind; diese Polyaminoamide können mit einem Epihalohydrin, Diepoxid, Dianhydrid, ungesättigten Dianhydrid, zweifach ungesättigten Derivat, Bis-halohydrin, Bis-azetidinium, Bis-haloacyldiamin, Alkyl-bis-halogenid oder auch einem Oligomer vernetzt sein, das bei der Reaktion einer bifunktionellen Verbindung gebildet wird, die gegenüber einem Bis-halohydrin, Bis-azetidinium, Bis-haloacyldiamin, Alkyl-bis-halogenid, Epihalohydrin, Diepoxid oder zweifach ungesättigten Derivat reaktiv ist; wobei das Vernetzungsmittel in Mengenanteilen von 0,025 bis 0,35 mol pro Aminogruppe des Polyaminoamids verwendet wird; diese Polyaminoamide können alkyliert oder, wenn sie eine oder mehrere tertiäre Aminofunktionen enthalten, quaternisiert sein;
(5) Polyaminoamiden, die bei der Kondensation von Polyalkylenpolyaminen mit Polycarbonsäuren und anschließender Alkylierung mit bifunktionellen Stoffen entstehen;
(6) Polymeren, die durch Reaktion eines Polyalkylenpolyamins, das zwei primäre Aminogruppen und mindestens eine sekundäre Aminogruppe aufweist, mit einer Dicarbonsäure erhalten werden, die unter Diglycolsäure und aliphatischen, gesättigten Dicarbonsäuren mit 3 bis 8 Kohlenstoffatomen ausgewählt ist;
(7) Alkyldiallylamin-Cyclopolymeren oder Dialkyldiallylammonium-Cyclopolymeren;
(8) quartären Diammoniumpolymeren, die Wiederholungseinheiten der folgenden Formel enthalten: 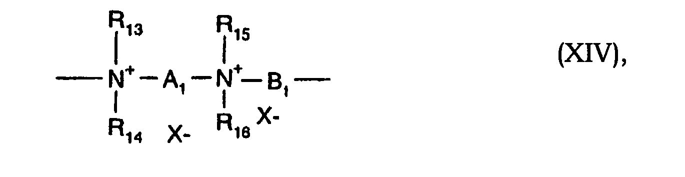 wobei in der Formel (XIV) bedeuten:
die Gruppen R₁₃, R₁₄, R₁₅ und R₁₆, die gleich oder verschieden sind, aliphatische, alicyclische oder arylaliphatische Gruppen mit 1 bis 20 Kohlenstoffatom oder niedere hydroxyalkylaliphatische Gruppen, oder die Gruppen R₁₃, R₁₄, R₁₅ und R₁₆ bilden gemeinsam oder unabhängig voneinander mit den Stickstoffatomen, an die sie gebunden sind, Heterocyclen, die gegebenenfalls ein von Stickstoff verschiedenes weiteres Heteroatom enthalten, oder R₁₃, R₁₄, R₁₅ und R₁₆ bedeuten eine geradkettige oder verzweigte C₁₋₆-Alkylgruppe, die mit Nitril, Ester, Acyl, Amid oder -CO-O-R₁₇-D oder -CO-NH-R17-D substituiert ist, wobei R₁₇ eine Alkylengruppe ist und D eine quartäre Ammoniumgruppe bedeutet;
A₁ und B₁ bedeuten Polymethylengruppen mit 2 bis 20 Kohlenstoffatomen, die geradkettig oder verzweigt, gesättigt oder ungesättigt vorliegen können und an die Hauptkette gebunden oder in der Hauptkette einen oder mehrere aromatische Ringe oder ein oder mehrere Sauerstoffatome, Schwefelatome oder die folgenden Gruppen enthalten können: Sulfoxid, Sulfon, Disulfid, Amino, Alkylamino, Hydroxy, quartäres Ammonium, Ureido, Amid oder Ester, und
X⁻ ein Anion, das von einer anorganischen oder organischen Säure abgeleitet ist;
A₁, R₁₃ und R₁₅ können mit den beiden Stickstoffatomen, an die sie gebunden sind, einen Piperazinring bilden; wenn A₁ eine geradkettige oder verzweigte, gesättigte oder ungesättigte Alkylen- oder Hydroxyalkylengruppe bedeutet, kann B₁ ferner auch eine Gruppe (CH₂)ₙ-CO-D-OC-(CH₂)ₙ- sein, worin D bedeutet:
a) einen Glycolrest der Formel -O-Z-O-, wobei Z eine geradkettige oder verzweigte Kohlenwasserstoffgruppe oder eine Gruppe bedeutet, die eine der folgenden Formeln entspricht:
-(CH2-CH₂-O)ₓ-CH₂-CH₂-
-[CH₂CH(CH₃)-O]_{y}-CH₂-CH(CH₃)-
worin x und y eine ganze Zahl von 1 bis 4 bedeuten und einen wohldefinierten und einzigen Polymerisationsgrad angeben oder eine beliebige Zahl von 1 bis 4 und einen mittleren Polymerisationsgrad bedeuten;
b) einen bis-sekundären Diaminrest, wie ein Piperazinderivat;
c) einen bis-primären Diaminrest der Formel -NH-Y-NH-, wobei Y eine geradkettige oder verzweigte Kohlenwasserstoffgruppe ist, oder die zweiwertige Gruppe -CH₂-CH₂-S-S-CH₂-CH₂-;
d) eine Ureylengruppe der Formel -NH-CO-NH-; vorzugsweise bedeutet X⁻ ein einwertiges anorganisches oder organisches Anion;
(9) quartären Polyammoniumpolymeren, die aus Einheiten der Formel (XVI) bestehen: 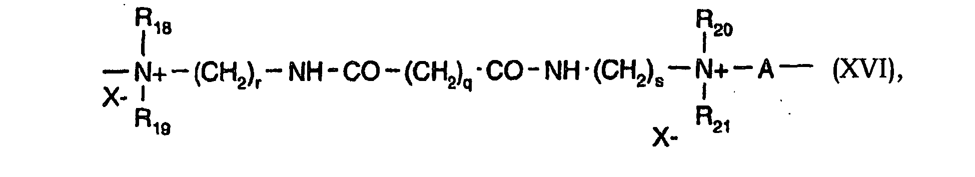 wobei in der Formel bedeuten:
die Gruppen R₁₈, R₁₉, R₂₀ und R₂₁, die identisch oder voneinander verschieden sind, ein Wasserstoffatom oder Methyl, Ethyl, Propyl, β-Hydroxyethyl, β-Hydroxypropyl oder -CH₂CH₂(OCH₂CH₂)ₚOH,
wobei p 0 oder eine ganze Zahl von 1 bis 6 bedeutet, mit der Maßgabe, dass R₁₈, R₁₉, R₂₀ und R₂₁ nicht gleichzeitig ein Wasserstoffatom bedeuten,
r und s, die gleich oder verschieden sind, bedeuten ganze Zahlen von 1 bis 6,
q bedeutet 0 oder eine ganze Zahl von 1 bis 34,
X ist ein Halogenatom,
A ist ein Dihalogenid oder bedeutet vorzugsweise -CH₂-CH₂-O-CH₂-CH₂-;
(10) quartären Polymeren von Vinylpyrrolidon und Vinylimidazol;
(11) Polyaminen, beispielsweise «POLYETHYLENGLYCOL (15) TALLOW POLYAMINE»;
(12) vernetzten Polymeren von Methacryloyloxyalkyl(C₁₋₄)trialkyl(C₁₋₄)ammoniumsalzen;
(13) Polyalkyleniminen, insbesondere Polyethyleniminen, Polymeren, die Vinylpyridin- oder Vinylpyridiniumeinheiten enthalten, Kondensaten von Polyaminen und Epichlorhydrin, quartären Polyureylenen und Chitinderivaten.

15. Zusammensetzung nach Anspruch 14, **dadurch gekennzeichnet, dass** die kationischen Polymere unter den kationischen Cyclopolymeren, kationischen Polysacchariden, quartären Polymeren von Vinylpyrrolidon und Vinylimidazol und deren Gemischen ausgewählt sind.

16. Zusammensetzung nach Anspruch 15, **dadurch gekennzeichnet, dass** das Cyclopolymer nach den Homopolymeren von Diallyldimethylammoniumchlorid und Copolymeren von Diallyldimethylammoniumchlorid und Acrylamid ausgewählt ist.

17. Zusammensetzung nach Anspruch 15, **dadurch gekennzeichnet, dass** die kationischen Polysaccharide unter den Stärken, die mit einem 2,3-Epoxypropyltrimethylammoniumsalz modifiziert sind, Guargummen, die mit einem 2,3-Epoxypropyltrimethylammoniumsalz modifiziert sind, und Hydroxyethylcellulosen ausgewählt sind, die mit einem mit einer Trimethylammoniumgruppe substituierten Epoxid umgesetzt wurden.

18. Zusammensetzung nach Anspruch 15, **dadurch gekennzeichnet, dass** die quartären Polymere von Vinylpyrrolidon und Vinylimidazol unter den Copolymeren von Vinylpyrrolidon und Methylvinylimidazoliumsalzen ausgewählt sind.

19. Zusammensetzung nach einem der vorhergehenden Ansprüche, **dadurch gekennzeichnet, dass** die Zusammensetzung mindestens ein kationisches Polysaccharid und mindestens ein quartäres Polymer von Vinylpyrrolidon und Vinylimidazol enthält.

20. Zusammensetzung nach einem der vorhergehenden Ansprüche, **dadurch gekennzeichnet, dass** die Zusammensetzung mindestens ein Diallyldiammoniumchloridhomopolymer und mindestens ein quartäres Polymer von Vinylpyrrolidon und Vinylimidazol enthält.

21. Zusammensetzung nach einem der vorhergehenden Ansprüche, **dadurch gekennzeichnet, dass** jedes kationische Polymer in einer Konzentration von 0,001 bis 20 Gew.-%, bezogen auf das Gesamtgewicht der Zusammensetzung, und vorzugsweise 0,01 bis 10 Gew.-% enthalten ist.

22. Zusammensetzung nach einem der Ansprüche 1 bis 21, **dadurch gekennzeichnet, dass** die nichtionischen verdickenden Polymere ausgewählt sind unter:
- nichtionischen Homopolymeren und Copolymeren, die Monomere mit ethylenisch ungesättigter Bindung vom Typ Ester und/oder Amid enthalten;
- Homo- oder Copolymeren von Vinylpyrrolidon;
- Polysacchariden.

23. Zusammensetzung nach Anspruch 22, **dadurch gekennzeichnet, dass** die nichtionischen Homopolymere und Copolymere, die Monomere mit ethylenisch ungesättigter Bindung vom Typ Ester und/oder Amid enthalten, unter den Polyacrylamiden, Methylmethacrylat/Ethylenglycoldimethacrylat-Copolymeren, Butylmethacrylat/Methylmethacrylat-Copolymeren und Polymethylmethacrylaten ausgewählt sind.

24. Zusammensetzung nach Anspruch 22, **dadurch gekennzeichnet, dass** die Homo- oder Copolymere von Vinylpyrrolidon insbesondere unter den vernetzten Vinylpyrrolidon-Homopolymeren ausgewählt sind.

25. Zusammensetzung nach Anspruch 22, **dadurch gekennzeichnet, dass** die Polysaccharide unter den Glucanen, modifizierten oder nicht modifizierten Stärkeverbindungen, Amylose, Amylopektin, Glycogen, Dextranen, Cellulosen und deren Derivaten (Methylcellulosen, Hydroxyalkylcellulosen, Ethylhydroxyethylcellulosen), Mannanen, Xylanen, Ligninen, Arabanen, Galactanen, Galacturonanen, Chitin, Chitosanen, Glucoronoxylanen, Arabinoxylanen, Xyloglucanen, Glucomannanen, Pektinsäuren und Pektinen, Arabinogalactanen, Carrageeninen, Agar-Agar, Gummi arabicum, Tragant, Ghatti-Gummi, Karaya-Gummi, Johannisbrotkemmehl, Galactomannanen, wie Guargummen und deren nichtionischen Derivaten (Hydroxypropylguar) und deren Gemischen ausgewählt sind.

26. Zusammensetzung nach einem der vorhergehenden Ansprüche, **dadurch gekennzeichnet, dass** das nichtionische verdickende Polymer in einer Konzentration von 0,001 bis 20 Gew.-%, bezogen auf das Gesamtgewicht der Zusammensetzung, und vorzugsweise 0,01 bis 10 Gew.-% enthalten ist.

27. Zusammensetzung nach einem der vorhergehenden Ansprüche, **dadurch gekennzeichnet, dass** sie ferner mindestens ein zusätzliches Konditioniermittel enthält.

28. Zusammensetzung nach Anspruch 27, **dadurch gekennzeichnet, dass** das Konditioniermittel unter den Siliconen, Carbonsäureestern mit mindestens 12 Kohlenstoffatomen, pflanzlichen Ölen, Mineralölen, synthetischen Ölen und deren Gemischen ausgewählt ist.

29. Zusammensetzung nach einem der vorhergehenden Ansprüche, **dadurch gekennzeichnet, dass** das kosmetisch akzeptable wässrige Medium aus Wasser oder einem Gemisch von Wasser und einem kosmetisch akzeptablen Lösungsmittel besteht.

30. Zusammensetzung nach Anspruch 29, **dadurch gekennzeichnet, dass** das kosmetisch akzeptable Lösungsmittel unter den niederen C₁₋₄-Alkoholen, Alkylenglycolen, Polyolethern, C₅₋₁₀-Alkanen, Aceton, Methylethylketon, C₁₋₄-Alkylacetaten, Dimethoxyethan, Diethoxyethan und deren Gemischen ausgewählt ist.

31. Zusammensetzung nach einem der vorhergehenden Ansprüche, **dadurch gekennzeichnet, dass** sie ferner Zusatzstoffe enthält, wie anionische, nichtionische oder amphotere Polymere, nicht polymere Verdickungsmittel, wie Säuren oder Elektrolyte, Trübungsmittel, Perlglanzstoffe, Vitamine, Provitamine wie Panthenol, Wachse wie pflanzliche Wachse, natürliche oder synthetische Ceramide, Parfums, Farbmittel, organische oder anorganische Partikel, Konservierungsmittel und pH-Stabilisatoren.

32. Zusammensetzung nach einem der vorhergehenden Ansprüche, **dadurch gekennzeichnet, dass** sie als Haarwaschmittel, Konditioner, Zusammensetzung für dauerhafte Verformungen, Entkräuselungen, Färbungen oder Entfärbungen der Haare oder Zusammensetzung, die ausgespült wird und zwischen den beiden Schritten eine dauerhafte Verformung oder Entkräuselung aufgetragen wird, vorliegt.

33. Zusammensetzung nach einem der vorhergehenden Ansprüche, **dadurch gekennzeichnet, dass** sie als Konditioner vorliegt, der ausgespült wird.

34. Verwendung einer Zusammensetzung nach einem der vorhergehenden Ansprüche zur Konditionierung oder die Pflege von Keratinsubstanzen.

35. Verfahren zur Behandlung von Keratinsubstanzen, wie Haaren, **dadurch gekennzeichnet, dass** es darin besteht, auf die Substanzen eine wirksame Menge einer kosmetischen Zusammensetzung nach einem der Ansprüche 1 bis 33 aufzutragen und gegebenenfalls zu spülen.

36. Verwendung einer Zusammensetzung nach einem der Ansprüche 1 bis 33, um den Haaren Glanz zu verleihen.

37. Verwendung einer Zusammensetzung nach einem der Ansprüche 1 bis 33, um dem Haar Geschmeidigkeit zu geben.
